(19) Europäisches Patentamt
European Patent Office
Office européen des brevets

(11) EP 1 565 573 B1

(12) **EUROPEAN PATENT SPECIFICATION**

(45) Date of publication and mention of the grant of the patent:
**21.03.2007 Bulletin 2007/12**

(21) Application number: **03775414.0**

(22) Date of filing: **19.11.2003**

(51) Int Cl.:
***C12Q 1/68*** *(2006.01)* *C12N 15/11* *(2006.01)*

(86) International application number:
**PCT/FI2003/000888**

(87) International publication number:
**WO 2004/046379 (03.06.2004 Gazette 2004/23)**

# (54) NUCLEIC ACID PROBES AND BROAD-RANGE PRIMERS FROM REGIONS IN TOPOISOMERASE GENES, AND METHODS IN WHICH THEY ARE USED

NUKLEINSÄURESONDEN UND BREITE PRIMERBEREICHE AUS TOPOISOMERASEGENBEREICHEN UND VERFAHREN MIT DEREN ANWENDUNG

SONDES D'ACIDE NUCLEIQUE ET AMORCES A LARGE SPECTRE PROVENANT DE REGIONS DE GENES DE LA TOPOISOMERASE, ET PROCEDES UTILISANT CELLES-CI

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **19.11.2002 FI 20022064**

(43) Date of publication of application:
**24.08.2005 Bulletin 2005/34**

(73) Proprietor: **Mobidiag Oy**
**00290 Helsinki (FI)**

(72) Inventors:
- **ROTH, Stina**
**FIN-00400 Helsinki (FI)**
- **JALAVA, Jari**
**FIN-20100 Turku (FI)**
- **NIKKARI, Simo**
**FIN-20700 Turku (FI)**

(74) Representative: **Lax, Monica Ingeborg et al**
**Kolster Oy Ab,**
**Iso Roobertinkatu 23,**
**P.O. Box 148**
**00121 Helsinki (FI)**

(56) References cited:

| | |
|---|---|
| **EP-A1- 0 965 636** | **EP-A2- 0 935 003** |
| **EP-A2- 1 098 003** | **WO-A1-00/61796** |
| **WO-A2-00/52203** | **WO-A2-01/36683** |
| **WO-A2-99/50458** | **GB-A- 2 364 054** |
| **US-A- 5 645 994** | **US-A- 6 087 104** |

- **SATOSHI YAMAMOTO ET AL.: 'PCR Amplification and Direct Sequencing of gyrb Genes with Universal Primers and their Application to the Detection and Taxonomic Analysis of Pseudomonas putita Strains' APPLIED AND ENVIRONMENTAL MICROBILOGY vol. 61, no. 3, March 1995, pages 1104 - 1109, XP002209784**
- **DATABASE WPI Week 200170, Derwent Publications Ltd., London, GB; Class B04, AN 2001-610077, XP002977257 & JP 2001 245677 A (KAIYO BIOTECHNOLOGY KENKYUSHO KK) 11 September 2001**
- **DATABASE WPI Week 200027, Derwent Publications Ltd., London, GB; Class C06, AN 2000-306710, XP002977258 & JP 2000 060570 A (CANON KK) 29 February 2000**
- **MASAO FUKUSHIMA ET AL.: 'Phylogenetic Analysis of Salmonella, Shigella, and Escherichia Coli Strains on the Basis of the gerB Gene Sequence' JOURNAL OF CLINICAL MICROBIOLOGY vol. 40, no. 8, August 2002, pages 2779 - 2785, XP002977259**
- **WAI MUN HUANG: 'Bacterial Diversity Based on Type II DNA Topoisomerase Genes' ANNUAL REVIEW OF GENETICS vol. 30, 1996, pages 79 - 107, XP000891519**
- **MASAO FUKUSHIMA: 'Detection and Identification of Mycobacterium Species Isolates by DNA microarray' JOURNAL OF CLINICAL MICROBIOLOGY vol. 41, no. 6, June 2003, pages 2605 - 2615, XP002977260**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the invention

The invention relates to nucleic acid probes and to broad-range primers that are useful in the identification of bacterial species and the diagnosis of bacterial infections. Especially, the invention relates to specific nucleic acid probes that originate from hyper-variable regions situated near the conserved sequences of topoisomerase genes of infection-causing bacteria. The invention also relates to broad-range primers originating from the conserved regions of topoisomerase genes. In addition, the invention relates to the use of these nucleic acid probes and broad-range primers in the diagnosis of bacterial infections as well as to diagnostic methods in which these nucleic acid probes and broad-range primers are used.

### Background of the invention

Respiratory tract infections are the most common cause for physician office visits. In Finland, approximately 13 pneumonia cases per 1000 inhabitants are diagnosed annually. Acute maxillary sinusitis and otitis are diagnosed even more frequently. For example, it is estimated that otitis accounts annually for about 200 000 cases in Finland (mainly diagnosed in children). Respiratory tract infections burden the health care system and cause significant expenses for the society. The exact costs attributable to respiratory tract infections are difficult to calculate, because the majority of these costs consist of so-called indirect costs, such as absence from work (Rautakorpi *et al.*, Scandinavian Journal of Infectious Diseases. 33(12): 920-6, 2001, Infektiotaudit, ed. Eskola, Huovinen ja Valtonen 1998). Worldwide, respiratory tract infections are responsible for the deaths of millions of people, mainly children.

In addition to viruses, respiratory tract infections are caused by a variety of bacterial species: *Streptococcus pyogenes* (group A streptococcus) is an important causative agent of tonsillitis. The risk of severe complications, such as peritonsillar abscesses, is connected to untreated tonsillitis. Furthermore, sequelae of tonsillitis caused by *S. pyogenes* include rheumatic fever and glomerulonephritis that are severe, even fatal diseases. The most important causative agents of pneumonia in outpatients are *Streptococcus pneumoniae* (pneumococcus), *Mycoplasma pneumoniae,* and *Chlamydia pneumoniae* from which pneumococcus is the most common and serious pathogen causing pneumonia. Less frequent pneumonia-causing bacterial species are *Legionella pneumophila* and *Coxiella bumetii. Mycobacterium tuberculosis,* on the other hand, causes pulmonary tuberculosis. Furthermore, pneumonia caused by rare *Mycobacterium* and *Nocardia* species is diagnosed in immunodeficient patients. In addition to pneumococcus, causative agents of maxillary sinusitis and inflammation of the middle ear (otitis media) include *Haemophilus influenzae* and *Moraxella catarrhalis.* Also *Alloiococcus otitidis* causes otitis, although less frequently.

At the moment the diagnosis of bacterial respiratory tract infections is mainly based on bacterial culture testing. Bacterial culture testing is, however, relatively slow, and diagnostic methods based on cultivation usually give results only days, sometimes up to weeks after sampling. Furthermore, cultivation of bacteria is not always successful under laboratory conditions. This can be a consequence of either the fact that the culture method used is not applicable for the bacterial species in question or the fact that the patient has received antimicrobial therapy before the sample is taken. In the diagnosis of pharyngitis, rapid methods based on the antigen detection are good supplementary methods to bacterial culture testing, but they work only with a limited number of pathogens (group A streptococcus). In the diagnostics of some bacterial infections (e.g., C. *pneumoniae* and *M. pneumoniae*) serological methods can also be used, but these methods give results only after several weeks from the onset of infection. Thus, they are not helpful in the acute treatment of a patient.

Molecular methods based on amplification and hybridization of nucleic acids attempt to solve the above-mentioned problems of bacterial culture testing. With the help of these methods the pathogen is simultaneously detected and identified, resulting in more rapid diagnostics and obviating the need for additional culture tests. Also, antibiotics do not interfere with molecular methods to such a degree as with culture testing.

One molecular method used in bacterial diagnostics is the so-called broad-range PCR (polymerase chain reaction) that is based on the use of broad-range primers. At the moment the most common broad-range PCR methods are based on the use of primers that recognize conserved DNA sequences of bacterial chromosomal genes encoding ribosomal RNA (16S rDNA/rRNA or 23S rDNA/rRNA). In bacterial identification based on broad-range PCR the actual identification step is carried out by cloning and sequencing the amplified PCR product. (See e.g., EP- B1 613 502, U.S. Patent No. 6,001, 564, and U.S. Patent Application Serial No. 0020055101).

The broad-range bacterial PCR method has to some extent been applied to clinical bacterial diagnostics, although it is best suited for the identification of bacterial and fungal species from culture isolates, and for this purpose commercial tests, such as MicroSeq (Applied Biosystems), have also been developed. However, these tests are not widely used, because sequencing of PCR products is time-consuming and labor-intensive. Also, the assays themselves and the necessary equipment, such as sequencing instruments, are expensive, and performing the tests requires specially trained personnel. Furthermore, in polymicrobial infections extensive cloning experiments may be needed.

Another method used to some extent in bacterial diagnostics is multiplex PCR, which is based on the use of several specific oligonucleotides pooled in one reaction mixture. Hendolin *et al.* (Journal of Clinical Microbiology, 35: 11, 1997) used the multiplex PCR method when identifying pathogens causing otitis media.

Although the multiplex PCR procedure is relatively sensitive and rapid, it has some disadvantages. It is known that shorter DNA fragments are amplified more efficiently than longer DNA fragments. Therefore, if the same specimen includes two different bacterial species, the bacterial DNA of the shorter fragment is likely to be amplified more efficiently, which affects the sensitivity of the procedure. Furthermore, with multiplex PCR it is possible to identify only a few bacterial species simultaneously, because it is practically impossible to design for example over a dozen specific primer pairs so that they would be functional under the same PCR conditions. Therefore, multiplex PCR is not applicable, e.g., to the diagnostics of, e.g., respiratory tract infections in which more than ten clinically important pathogens are known.

Bacteria of so-called normal flora can also cause problems in microbial diagnostics based on multiplex PCR. The genome of only a few dozen bacterial species has been wholly mapped and most of these bacterial species are known disease causative agents. Thus, there is very little information about the bacteria of normal flora and their DNA sequences. This is why the design of species-specific PCR primers and bacterial diagnostics based solely on PCR amplification are almost impossible.

The use of ribosomal RNA also includes some drawbacks. Distinguishing the related bacterial species from each other with the help of rRNA molecules is difficult, because the sequences of these molecules do not contain enough variable regions when they are compared with each other. And even if differences between various bacterial species may be found, these variable sites are generally divided across the whole rRNA molecule (e.g., the length of 16S rRNA is about 1500 nucleotides), which limits the use of molecular methods for diagnostics. In practice, the related bacterial species can thus be distinguished from each other only by sequencing the whole rRNA encoding gene. However, even this approach is not always sufficient to distinguish bacterial species from each other.

## Brief description of the invention

The purpose of the present invention is to provide tools and methods, which are useful in bacterial diagnostics of infectious diseases, especially those causing respiratory tract infections, but which lack the drawbacks of bacterial diagnostics described above. In particular, the purpose of the invention is to provide novel tools and methods, which are useful in bacterial diagnostics based on molecular methods, the tools and methods being sensitive, effective, and species-specific, and being capable of identifying only the desired bacterial species. A further purpose of the invention is to provide methods, by which it is possible to diagnose infectious bacteria, especially those that cause respiratory tract infections, substantially faster than previously possible, whereby correct and effective antimicrobial therapy can be prescribed to the patient at an earlier stage of the infection so that the duration of the infection becomes shorter and the risk of potentially harmful, even life-threatening complications is reduced.

The present invention provides bacterial species-specific oligonucleotide probes that originate from hyper-variable regions situated near the conserved regions of genes encoding topoisomerases, especially the *gyrB*/*parE genes,* these hyper-variable regions differing in the base sequences significantly in various bacterial species. With these bacterial species-specific probes the genome of multiple infection-causing bacteria can be simultaneously detected and identified.

The invention also provides broad-range or broad-range primers that originate from the conserved regions of genes encoding topoisomerases, especially the *gyrB*/*parE* genes, and that efficiently amplify DNA of infection-causing bacteria even from clinical specimens, which include large amounts of foreign (non-bacterial) DNA.

Furthermore, the present invention provides simple, rapid, sensitive, and specific methods that overcome the drawbacks of the prior art. With these methods clinically important bacterial species can be reliably identified and diagnosed from clinical specimens or bacterial cultures.

The present invention relates to oligonucleotide probe sequences that hybridize under normal hybridization conditions with sequences of hyper-variable regions situated near the conserved sequences of topoisomerase genes of bacteria that cause infections, especially infections of the respiratory tract, and comprise any one of the sequences identified with SEQ. ID. NO. 1 to 69, and/or reverse or complementary sequences thereof, or functional fragments thereof.

Examples of bacterial species that cause infections, especially infections of the respiratory tract, include *Streptococcus pneumoniae, Streptococcus pyogenes, Chlamydia pneumoniae, Mycoplasma pneumoniae, Haemophilus influenzae, Staphylococcus aureus, Pseudomonas aeruginosa, Neisseria gonorrhoeae, Escherichia coli, Moraxella catarrhalis, Legionella pneumophila,* and *Fusobacterium necrophorum,* and examples of genes encoding topoisomerase are the gene encoding *gyrB* and *parE* proteins.

Preferably the length of oligonucleotide probe sequence of the invention is 15 - 30 nucleic acids, more preferably 20 to 30 and most preferably 21 to 25 nucleic acids.

The present invention also relates to the use of the above-mentioned oligonucleotide probe sequences in the detection, identification, or classification of bacterial species.

The present invention further relates to a mixture of oligonucleotide probes, which comprises any combination, and preferably all the sequences identified with SEQ. ID. NO. 1 to 69, and/or their reverse or complementary sequences, or functional fragments of the afore-mentioned sequences. In one preferred embodiment, the desired mixture of probes has been attached to a solid support. Preferably, an oligonucleotide probe mixture that comprises all sequences identified with SEQ. ID. NO. 1 to 69, and/or their reverse or complementary sequences, has been attached onto a solid support.

The present invention further relates to a novel mixture of DNA primers that comprises sequences that hybridize with the sequences of conserved regions of genes encoding topoisomerases, especially with the genes encoding the gyrB and/or parE proteins of bacteria causing respiratory tract infections, and which comprise sequences identified with SEQ. ID. NO. 76 or 77 or their functional fragments. The present invention also relates to the use of the above mentioned mixture of primers in the amplification of topoisomerase genes, especially genes encoding the *gyrB* and *parE* proteins.

Furthermore, the present invention relates to a diagnostic method for detecting and identifying bacteria causing respiratory tract infections in a clinical specimen, which comprises

a) amplifying DNA isolated from the clinical specimen using the above- mentioned mixture of primers,
b) contacting the amplified DNA with a desired combination of the above-mentioned probes under hybridization conditions, and
c) detecting the formation of a possible hybridization complex.

One preferred embodiment of the method of the invention comprises amplifying the DNA isolated from the clinical specimen by polymerase chain reaction, and contacting the amplified DNA with the bacterial species-specific oligonucleotide probes attached to the solid support.

In one preferred embodiment of the method of the invention, a suitably labeled nucleotide is used in the amplification of DNA isolated from the clinical specimen in order to generate a detectable target strand.

In another preferred embodiment of the method of the invention, the amplified and possibly labeled target DNA is contacted with a solid support, preferably with treated glass on which all the species-specific oligonucleotide probes of the invention having sequences identified with SEQ. ID. NO. 1 to 69 and/or their reverse or complementary sequences have been attached.

In a further preferred embodiment of the method of the invention, the amplified and possibly labeled target DNA is contacted with a solid support, preferably with treated glass, on which the specific oligonucleotide probes of the invention of one specified bacterium or a few specified bacteria causing respiratory tract infections have been attached, said specific oligonucleotide probes having corresponding sequences identified with SEQ. ID. NO. 1 to 7, 8 to 14, 15 to 16, 17 to 20, 21 to 25, 26 to 29, 30 to 33, 34 to 38, 39 to 42, 43 to 47, 48 to 50, 51 to 55, 56 to 60, 61 to 65, and 66 to 69 from Table 4A and 4B and/or their reverse or complementary sequences thereof, or any suitable combination thereof.

The present invention further relates to a diagnostic kit for use in the diagnosis of infection-causing bacteria, especially those causing respiratory tract infections, comprising

a) a DNA primer mixture of the invention as defined above,
b) a mixture of bacterial species-specific oligonucleotide probe sequences, optionally attached on a solid support, of the invention as defined above,
c) positive and optionally negative control probe sequences, and optionally
d) reagents required in the amplification, hybridisation, purification washing, and/or detection steps.

**Brief description of figures**

Figure 1 shows examples of hyper-variable *gyrB* gene regions that are limited by the conserved sequences (*Haemophilus influenzae* and *Moraxella catharralis*). The conserved regions have been marked with gray boxes, and they act as the annealing sites of broad-range *gyrB*/*parE* primers. The hyper-variable region, from which bacterial species-specific probes have been designed, is between these conserved sequences (underlined sites).

Figure 2 shows an example of the results of a hybridization on the surface of the microarray. The amplified DNA of the *gyrB* gene isolated from a culture isolate of *S. aureus* was used as the target strain (asymmetric Cy-5-dCTP labeled PCR product). The glass slide contained four specific *gyrB* oligonucleotide probes (Table 4A: oligonucleotide probes 21 to 24) that all bound specifically to the target strain, *S. aureus*. In addition, all 5 positive control oligonucleotides gave a detectable signal. The target strand, *S. aureus* did not bind to other oligonucleotide probes on the glass slide.

Figure 3 shows the PCR amplification results from Example 7, in which known primers described as specific for *Streptococcus pneumoniae* were compared in testing the bacterial species of *Streptococcus* genera and known primers described as specific for *Moraxella catarrhalis* were compared in the testing the bacterial species of *Moraxella* genera. The results show, however, that the primers in question are not species-specific, but they also amplify the bacteria of normal flora.

## Detailed description of the invention

The present invention is based on studies, which attempted to find more specific alternatives for the use of ribosomal RNA in the diagnostics of infection-causing bacteria. The study was focused on other genes that are vital for bacteria. The topoisomerases gyrase B (*gyrB* or topoisomerase II) and *parE* proteins (the other subunit of topoisomerase IV) are important molecules for bacteria, because they are needed in the packing of DNA. Certain DNA sequence fragments of these molecules have remained almost unchanged, i.e. conserved, during evolution. In this context the terms "a conserved region" or "conserved regions" refer to a region or regions of topoisomerase genes or proteins, whose nucleotide sequence or equivalently the amino acid sequence has remained nearly unchanged between different bacterial species causing respiratory tract infections. Usually these conserved regions are the most important regions for the functioning of the protein. *GyrB*/*ParE* molecules are, however, not as conserved as ribosomal RNA molecules. Because the molecules in question are proteins, the genes encoding these proteins include more differences at the nucleic acid level, due to the nature of their genetic code, than the genes encoding structural RNA molecules (e.g., 16S rRNA molecules). *GyrB*/*ParE* molecules as a whole have neither remained as unchanged as the structural RNA molecules during the evolution: short DNA fragments can be found in *gyrB*/*parE* molecules where the differences between various bacterial species great (including closely related bacterial species) are so great that these DNA sequences can be considered as species-specific. These sequences are hyper-variable sequences, which in this context refer to DNA sequences of the topoisomerase genes which differ in the nucleotide base sequence between different bacteria to an extent affording bacterial species-specificity and which are situated near the conserved sequences of the topoisomerase genes and optionally limited or marked out by the conserved sequences.

The above-mentioned features were utilized in the design of species-specific oligonucleotides for bacterial strains. In the design of species-specific probes (oligonucleotides) (Tables 4A and 4B) a planning strategy based on alignment was used. The *gyrB* or *parE* genes of target bacterial species were aligned with the corresponding genes of the reference bacteria. The sequences were obtained from the EMBL public sequence database or, in the cases where such sequences were not available in public databases, they were produced by cloning the *gyrB*/*parE* gene sequence fragments from the bacterial species. The sequences were produced by amplifying the desired *gyrB*/*parE* DNA fragment from bacterial culture isolates. The desired DNA sequence fragment was then cloned and sequenced. An example of the hyper-variable *gyrB* gene region of two bacterial species, *Haemophilus influenzae* and *Moraxella catharralis,* that is bounded by conserved gene regions is presented in Figure 1. Conserved regions have been marked with gray boxes, and they act as the annealing sites of broad-range *gyrB*/*parE* primers. The hyper-variable region, to which species-specific probes have been designed, is between these conserved sequences (underlined sites).

Alignment of the sequences was performed with the BioEdit program and the ClustalW alignment algorithm. The consensus sequence of the alignments was calculated and the suitably conserved regions were identified manually. These regions refer to sequence fragments that are conserved in the genes of the target bacterial species but are not found, at least entirely, in the genes of the reference bacterial species. Oligonucleotide sequences with the suitable length (e.g., 21 - 25 nucleotides) were selected from these areas for comparison analyses. The selected oligonucleotide sequences were compared to the EMBL prokaryotic sequence database using the FastA algorithm program. The oligonucleotide sequences having at least two mismatches when compared to *gyr*B/*par*E sequences of non-target bacterial species were chosen for further analyses. Melting temperatures (Tm) of oligonucleotides were calculated and the formation of hairpin structures was examined. The oligonucleotides without strong secondary structures and with Tm higher than 45 °C were selected for specificity testing. The specificity of the oligonucleotide probes was tested in laboratory conditions both with pure DNA samples isolated from various bacterial species (Example 3, Table 3) and with clinical patient samples (Example 6, Table 5).

The oligonucleotide probes of the present invention comprise the sequences identified with SEQ. ID. NO. 1 to 69, or their reverse or complementary sequences. They can be of different length, and only the desired species-specificity and functionality of these oligonucleotide probes determine their suitable length in hybridization reactions. The length of the oligonucleotide probes is generally 15 to 30, preferably 20 to 30 and most preferably 21 to 25 nucleotides. Furthermore, the probes can be modified in different ways (e.g., modified nucleotides, such as inosine, can be included). Also, various chemical compounds or groups (e.g. amino groups) or other molecules, such as labels necessary for the detection, can be attached to the probes, or they can be entirely unmodified. The sequences of the preferred bacterial species-specific probes and their specificities are presented in Tables 4A and 4B, and they have sequences identified with SEQ. ID. NO. 1 to 69. Naturally, reverse and complementary sequences of these oligonucleotide sequences are equally suitable, as is obvious to a person skilled in the art. Similarly, functional fragments of the previously mentioned oligonucleotide sequences are useful as probes provided that species-specificity remains unchanged.

For designing the PCR primers, amino acid sequences of *gyrB* proteins of bacterial species selected from different fylogenetic groups (Table 2) were aligned with the BioEdit program using the ClustalW alignment algorithm. In alignment studies many conserved regions were found and taken as the starting point for the broad-range primer design. The *parE* protein is related to the *gyrB* protein, and the above-mentioned conserved regions can be found in both of these genes in certain bacterial species (gram-positive bacteria). The conserved amino acid sequences were reverse-translated to the corresponding nucleic acid sequences. Depending on the nature of the genetic code there were several degenerated regions in the primer sequences. On the basis of the conserved sequences several primer pairs were designed and tested in laboratory (specificity and sensitivity testing). On the basis of the laboratory tests it was concluded that the amplification of pure bacterial DNA is successful with some of the primer pairs, and that the *gyrB*/*parE* genes of all bacteria can be amplified with these primers. The primers in question thus act as universal or broad-range primers for the bacteria.

One of the primer pairs proved to have superior sensitivity as compared to the others, and clinical specimens were tested with this primer pair. However, it turned out that bacterial DNA cannot be amplified with this primer pair from clinical specimens which contain large amounts of human DNA. For this reason the primer pair was redesigned, and new primer alternatives, in terms of degeneration, were sought, which would on one hand amplify DNA from clinical specimens, and on the other hand also retain a high specificity so that *gyrB*/*parE* proteins of all bacteria causing respiratory tract infections could be amplified. A functional primer pair is presented in Table 1. With this primer pair, all *gyr*B/*par*E genes of bacteria that are phylogenetically distant from each other (Table 3) can be amplified even in the presence of large amounts of human DNA.

**Table 1. *gyrB*/*parE* broad-range primers**

| Name of the primer | Sequence 5'->3' |
|---|---|
| gB1F | CGTCCWGGKATGTAYATHGG |
| gB2R | CCHACRCCRTGWAAWCCDCC |

In the primer sequences:

W represents base A or T,
K represents base G or T,
Y represents base C or T,
H represents base A or C or T,
R represents base A or G, and
D represents base A or G or T.

The primer mixtures, which consist of several different primer alternatives, are thus concerned. For example, in the case of primer gB1F the mixture includes primers, in which W represents A (adenine) or T (thymine).

According to the invention, specific probes can be used for identification of infection-causing pathogens, especially bacteria that cause respiratory tract infections, in any suitable method, by which hybridization can be demonstrated. These methods are well known among the persons skilled in the art and they can be performed both in a solution and on a solid support that binds DNA, such as on nitrocellulose or nylon membrane, or on glass.

In one preferred embodiment of the method of the invention, bacterial identification is performed using the DNA microarray technology. In this context, a DNA microarray or a DNA chip refers to a small substrate on which known nucleic acid sequences have been attached in a predetermined order. If the nucleic acid fragments attached to the microarray are shorter than 100 base pairs (generally around 20 - 30 base pairs), the microarray is called an oligonucleotide array.

In the method of the present invention the sample to be analyzed can be a bacterial culture, a tissue fragment, a secretion sample, such as a sputum or brush sample, a blood sample, or another suitable sample, obtained from a patient suspected of suffering of an infectious disease. Especially the sample to be analyzed is a secretion sample suitable for clinical diagnostic applications.

DNA is isolated from the sample to be analyzed with any conventional method, such as with commercial DNA isolation kits (e.g., High Pure PCR Template Preparation Kit, Roche; NucleoSpin, BD Biosciences Clontech; or QIAamp DNA Mini-kit, Qiagen) or with traditional extraction with phenol-chloroform or equivalent organic solvents, either manually or with special devices that are suitable for performing DNA isolation. Commercial kits are preferably used because of their general availability, rapidity, and repeatability.

In the method of the present invention the reagents used in DNA amplification can be any reagents that are conventionally used for the amplification of DNA, and are well known among the persons skilled in the art. Suitable and cost-effective reagents, which are commercially available, include different types of Taq DNA polymerases and buffers therefor (e.g., AmpliTaqGOLD, AmpliTaqLD, DyNAzyme, TaqPlus Precision, and HotStartTaq), nucleotides or pre-prepared mixtures of nucleotides (e.g., Sigma, Applied Biosystems, Amersham Biosystems), $MgCl_2$ (whereby a product

from the manufacturer of the Taq polymerase is generally used), and Cy5-dCTP (e.g., NEN LifeSciences, Amersham Biosciences).

In the method of the present invention cloning can be performed with any conventionally known method, for example by using commercially available cloning kits (e.g., Qiagen PCR Cloning Kit, QIAGEN or TOPO TA Cloning Kit, Invitrogen). Sequencing of the cloning products can be performed with any sequencer suitable for this purpose (e.g., Applied Biosystems, model 373A, 377, or 3100, or Bio-Rad Sequi-Gen GT), or the products can be sequenced manually. The sequences can be analyzed manually or with sequence analysis programs designed for this purpose (e.g., Applied Biosystems Sequencer or Vector NTI Suite Version 7, InforMax).

The equipment used for amplification can also be any suitable device (e.g., T1 Thermocycler, Biometra, or GenAmp PCR system 2700, Applied Biosystems). Practically all devices and equipment suitable for DNA amplification can be used, and amplification can also be performed manually by transferring reaction tubes from one temperature to another. In addition, amplification can be performed directly on a DNA microarray.

Purification of the PCR product can be performed with any commercial method (e.g., High Pure PCR Product Purification Kit, Roche; MicroSpin S-400, or S-300 HR Columns, Amersham Biosciences; or QIAquick PCR-purification-Kit, Qiagen) or using extraction with an organic solvent. The amplification product can also be used for the hybridization reaction as such without any further purification or extraction steps.

In order to form a single-stranded target strand any known digestion method can be used. These methods include, e.g., asymmetric PCR, exonuclease treatment, or the synthesis of a single-stranded target strand directly onto the microarray surface (e.g., matriXarray, Roche Applied Science). The invention also comprises applications in which a doublestranded PCR product can be used in the hybridization reaction. In the context of the present invention asymmetric PCR is the preferred method to generate a single-stranded target strand.

In the method of the present invention any suitable label can be used in order to produce a labeled target strand. Suitable labels include fluorescent labels (e.g., Cy5, Cy3, Cy2, TexasRed, FITC, Alexa 488, TMR, FluorX, ROX, TET, HEX), radioactive labels (e.g., $^{32}P$, $^{33}P$, $^{33}S$), and chemi-luminescent labels (e.g., HiLight Single-Color Kit). In the present invention the Cy5-dCTP fluorescent label (Amersham Biosciences) is preferred. The invention also comprises the applications in which no label is needed, such as those in which the detection of nucleic acids is based on electric impulse (e.g., the Motorola eSensor).

When hybridization takes place on a solid support, the probes used in hybridization can be attached onto the surface of the solid support by covalent or non-covalent binding. Alternatively, other chemical, electrochemical or equivalent attachment methods can be used. The substrate or support, on which the probes are attached, can be manufactured from glass, plastic, metal, nylon, nitrocellulose, polyacrylamide, silicon, or a combination of these materials, and the size of the substrate can vary from a couple of millimeters to a few centimeters. The surface of the substrate used can be treated with aminosilane or any other suitable surface treatment, such as epoxysilane, or alternatively a substrate that does not require any separate surface treatment can be used. A preferred substrate for the oligonucleotide probes is a microscopic glass slide treated with aminosilane (e.g., Genorama, Asper Biotech Ltd., Estonia).

The probes can be printed onto the surface of the microarray support with any commercially available arrayer that is suitable for this purpose (e.g., Qarray-mini arraying system, Lucidea Array Spotter, OmniGrid, or GeneMachines arrayer), or they can be pipetted manually onto the surface. Alternatively, the probes can be synthesized directly onto the surface by using photolithography.

The hybridization mixture used in hybridization can be different in its composition than what has been presented later in the working Examples, for example, the salt composition and/or the concentration can vary (e.g., 2-4xSSC or SSPE), or commercially available hybridization solutions can be used (e.g., ArrayHyb, Sigma). In addition, denaturing or stabilizing additives (e.g., formamide, DMSO, i.e. dimethyl sulfoxide) or substances that decrease non-specific binding (e.g., BSA, i.e. bovine serum albumin, or ssDNA, i.e. salmon sperm DNA) can be used in the hybridization mixture. Hybridization can be carried out in various hybridization temperatures (generally between 40-70°C), and the time needed to perform hybridization can vary, depending on the application, from a few minutes to one day. Instead of a water bath, hybridization can be carried out, e.g., in an incubator or in a special hybridization device (e.g., GeneTAC HybStation or Lucidea Slidepro Hybridizer). The post-hybridization washing steps can vary in their duration, volume, temperature, and in the composition of the washing solution, and can therefore differ from the exemplified method. The washing steps of microarray slides can also be performed with a separate device. In some cases a washing step is not necessary, because the microarray slide can be analyzed immediately after hybridization. In a preferred method a +57°C water bath is a suitable hybridization condition. The glass slides were hybridized for 12-16 hours in these conditions.

Microarrays or chips can be analyzed with any equipment or reader applicable for this purpose (e.g., GeneTAC UC4, GenePix Personal 4100A, or Agilent DNA Microarray Scanner). If the target strand has been marked with a fluorescent label, analysis can also be performed, e.g., with a fluorescent microscope. If a radioactive label has been used, the chip or membrane can be analyzed with autoradiography. If hybridization has been carried out on the surface of an electronic microarray and the analysis is thus based on electronic detection, the microarray can be analyzed with special equipment designed for this purpose.

The genome of only a few dozen bacterial species has been entirely mapped, and most of these bacterial species are known pathogens. Thus, there is very little information about normal bacterial flora and the DNA sequences of these bacterial species. Therefore, the design of bacterial species-specific PCR primers and bacterial diagnostics based solely on PCR amplification are almost impossible.

The method of the present invention does not suffer from the problems of the prior art. The amplification step of the method of the present invention is very sensitive and a certain gene region (*gyrB*/*parE*) from phylogenetically different bacterial species were amplified efficiently with broad-range primers of the invention regardless of whether the bacterial species were gram-negative or gram-positive (Table 3). Furthermore, the PCR product is short (about 300 base pairs), which improves the effectiveness of the amplification reaction.

The bacterial species-specific probes have been designed for the *gyrB*/*parE* gene region that is considerably more variable than for example the 16S rRNA gene region, which has previously been used in bacterial diagnostics. Although bacterial species of normal flora are also amplified efficiently with broad-range primers used in the present method, no false positive reactions occur, because the probes of the invention are very bacterial species-specific and identify only those bacteria for which they have been designed. When hybridizing the target strand amplified from culture isolates of *Streptococcus pneumoniae* onto a glass slide, the target strand will hybridize only with probes specific for *Streptococcus pneumoniae* and with positive controls. On the other hand, when hybridizing the target strand amplified from bacteria of normal flora, e.g. *Streptococcus mitis,* the product will not attach to any pathogen probe; in this case only positive control probes will emit a signal (Example 7, Table 6). All specific oligonucleotide probes of the present invention were cross-tested with various bacterial species, including many bacterial species that belong to normal flora, and no cross reactions were found to take place (Figure 2, Table 6). Thus, the method of the present invention is considerably more sensitive and specific than the previously described methods of similar type.

The diagnostic kit of the invention can be used in the diagnosis of infection-causing bacteria, especially those causing respiratory tract infections. The kit comprises the DNA primer mixture of the invention as defined above in detail, any suitable and desirable mixture of bacterial species-specific oligonucleotide probe sequences, optionally attached on a solid support, of the invention as defined above in detail, suitable positive and optionally negative control probe sequences, and optionally reagents required in the amplification, hybridisation, purification, washing, and/or detection steps as discussed in detail above. A preferred diagnostic kit of the invention contains a DNA primer mixture comprising sequences identified with SEQ. ID. NO. 76 and 77, a chip containing the oliginucleotide prope sequences identified with SEQ. ID. NO. 1 to 69 attached thereon, positive and optionally negative controls, and optionally the reagents required to perform the method of the invention. In the following, the present invention is more precisely illustrated with the Examples. When describing the method, references have been made to different equipment, materials, temperatures, chemicals, or equivalents used in this application. These can naturally be varied in a suitable way in different applications of the invention. Therefore, the present invention and its embodiments are not limited to the Examples described below.

### Example 1. The design of PCR primers according to the invention

For the design of PCR primers, amino acid sequences of the *gyrB* proteins (GyrB) and its related protein ParE of various bacterial species from different phylogenetic groups (Table 2) were aligned with the BioEdit program using the ClustalW alignment algorithm. Several conserved gene regions were found in the alignment of GyrB. The same conserved regions were also found from the *parE* gene of the studied gram-positive bacterial species.

These conserved amino acid sequences were used as a starting point in the design of broad-range primers. First they were reverse-translated to the corresponding nucleic acid sequences. Because of the nature of the genetic code several degenerated sites were observed in these nucleic acid sequences. After this, based on the conserved sequences, various primer pairs were synthesized (ordered from Sigma-Genosys, England, www.sigma-genosys.co.uk) and tested for specificity and sensitivity. The specificity was tested by amplifying DNA isolated from bacterial species presented in Table 4 using the method described below in Example 4. The sensitivity of the primer pair was determined by analyzing the smallest concentration of DNA isolated from *H. Influenzae* culture isolate that could be amplified and detected with different primer pairs using the amplification and identification method described below in Example 4.

Based on these test results, it was noted that the amplification of pure bacterial DNA was successful for some the primer pairs, and with these primers the *gyrB*/*parE* genes of all studied bacterial species could be amplified. These primers functioned therefore as broad-range primers for bacteria. Sensitivity of the primers varied, and the primer pair with the highest sensitivity was used for studying the clinical specimens (otitis media samples). However, it was found out that this primer pair was not sufficiently sensitive, because it was not able to amplify bacterial DNA from clinical specimens that contained large amounts of human DNA.

For this reason new primer pairs that varied in terms of degeneration were synthesized (ordered from Sigma-Genosys, England, www.sigma-genosys.co.uk). The specificity and the sensitivity were studied by the previously described method, both with pure bacterial DNA and with DNA isolated from clinical specimens (Table 5). A functioning primer pair was the mixture of primers, which contains the sequences

CGTCCWGGKATGTAYATHGG (SEQ. ID. NO. 76) and
CCHACRCCRTGWAAWCCDCC (SEQ. ID. NO. 77),
which were named as gB1F (forward primer mixture) and gB2R (reverse primer mixture), respectively (Table 1), wherein
W represents base A or T,
K represents base G or T,
Y represents base C or T,
H represents base A or C or T,
R represents base A or G, and
D represents base A or G or T.

The conserved sequences of the first part of the *gyrB* and/or *parE* genes of all studied bacterial species were identified with this mixture of primers. It amplifies DNA from clinical samples, and has preserved sufficiently broad specificity, thus enabling the amplification of the *gyrB*/*parE* genes from all bacterial species causing respiratory tract infections (see Examples 6 and 7). In particular, this mixture of primers can be used to amplify the *gyrB*/*parE* genes of bacteria (Table 3) that are phylogenetically far from each other even in a situation where the sample includes large amounts of human DNA.

**Table 2. Bacteria whose amino acid sequences of *gyrB* proteins were used for the alignment.**

| Genus | Species |
|---|---|
| *Bacteroides* | *fragilis* |
| *Mycobacteriun* | *tuberculosis* |
| *Streptococcus* | *pneumoniae* |
| *Thermotoga* | *maritima* |
| *Chlamydia* | *pneumoniae* |
| *Chlamydia* | *thrachomatis* |
| *Borrelia* | *burgdorferi* |
| *Escherichia* | *coli* |
| *Neisseria* | *gonorrhoeae* |
| *Haemophilus* | *influenzae* |
| *Myxococcus* | *xanthus* |
| *Campylobacter* | *jejuni* |
| *Helicobacter* | *pylori* |
| *Bartonella* | *bacilliformis* |
| *Aquifex* | *aeolicus* |

**Table 3. Bacterial strains used in sensitivity and specificity testing of primers and probes.**

| Bacterial species | Supplier's code (type of sample) |
|---|---|
| *Hemophilus influenzae* | ATCC 51907D (DNA) |
| *Neisseria gonorrhoeae* | ATCC 53420D (DNA) |
| *Mycoplasma pneumoniae* | ATCC 15531 D (DNA) |
| *Legionella pneumophila* | ATCC 33152D (DNA) |
| *Pseudomonas aeruginosa* | ATCC 47085D (DNA) |
| *Moraxella catarrhalis* | DSM 9143 (bacterial species) |
| *Staphylococcus aureus* | DSM 20231 (bacterial species) |
| *Streptococcus pyogenes* | DSM 20565 (bacterial species) |
| *Streptococcus pneumoniae* | DSM 20566 (bacterial species) |
| *Fusobacterium necrophorum* | DSM 20698 (bacterial species) |
| *Escherichia coli* | DSM 30083 (bacterial species) |
| *Pseudomonas aeruginosa* | DSM 50071 (bacterial species) |
| *Streptococcus oralis* | DSM 20627 (bacterial species) |
| Streptococcus *mitis* | DSM 12643 (bacterial species) |
| *Haemophilus parainfluenzae* | DSM 8978 (bacterial species) |
| *Haemophilus ducreyi* | DSM 8925 (bacterial species) |

(continued)

| Bacterial species | Supplier's code (type of sample) |
|---|---|
| *Moraxella caviae* | ATCC 14659 (bacterial species) |
| *Pasteurella pneumotropica* | ATCC 13669 (bacterial species) |
| *Moraxella cuniculi* | ATCC 14688 (bacterial species) |
| *Chalmydia pneumoniae* | ATCC VR 1355 (bacterial species) |

ATCC = American Type Culture Collection
DSM = Deutsche Sammlung von Mikroorganismen und Zellkulturen

**Example 2. Production of new sequences required in the design of oligonucleotide probes as defined by the invention by cloning**

*GyrB* sequences of the bacterial species *Moraxella catarrhalis, Fusobacterium necrophorum, Legionella pneumophila, Streptococcus mitis, Streptococcus oralis,* and *Haemophilus parainfluenzae* that are not available in public sequence databases were synthesized according to the general method described below in order to design oligonucleotide probes of the invention.

First DNA is isolated from bacterial culture isolates using the QIAamp DNA Mini kit (Qiagen, Germany). When DNA has been isolated, the desired target strand used for cloning is amplified using symmetric (conventional) polymerase chain reaction (PCR). In the first step of amplification, the reaction mixture is prepared by mixing the DNA isolated from the sample, the broad-range bacterial primers (Example 1), and the other components needed in the amplification step. Thus, the reaction mixture (25 μl) used in cloning PCR contains 20 pmol of primer mixture gB2R, 20 pmol of primer mixture gB1F, 200 μM of each of dATP, dGTP, dTTP, and dCTP (Sigma, USA), 1 x Hot Start Taq PCR buffer (Qiagen, Germany), which includes $MgCl_2$ in order to achieve a final concentration of 2.8 mM, 7.5% DMSO (Amersham Pharmacia Biotech, USA), 2.5 U Hot Start Taq DNA polymerase (Qiagen, Germany), and 2.5 μl of isolated DNA.

The cloning PCR is carried out in a GenAmp PCR system 2700 thermal cycler (Applied Biosystems) using the following program: a 15 min denaturation step at 95°C, 40 cycles of 1 min at 95°C, 1 min at 50°C, 1 min at 72°C, and finally a 10 min extension step at 72°C. After the PCR has been performed, the success of amplification is verified by gel electrophoresis using a 2% agarose gel containing ethidium bromide.

Cloning is performed immediately after PCR using a TOPO TA Cloning Kit (Invitrogen, USA). The reaction mixture for cloning contains 4 μl of the PCR product, 1 μl of a salt solution (1.2 M NaCl, 0.06 M $MgCl_2$), and 1 μl of TOPO vector (pCR 4-TOPO), which are mixed together in an eppendorf tube. The mixture is incubated for 5 min at room temperature, after which the solution is transferred onto ice. After this chemical transformation is performed, in which 2 μl of cooled cloning mixture is transformed into 50 μl of competent TOPO10 *E. coli* cells. The transformed cells are incubated for 10 min on ice. In the next stage, a heat-shock treatment is performed. The tube containing the cells is transferred to a 42°C water bath for 30 s. After this the tube is immediately transferred onto ice and 250 μl of SOC medium at room temperature is added (2% tryptone, 0.5% yeast extract, 10 mM Nacl, 2.5 mM KCl, 10 mM $MgCl_2$, 10 mM $MgSO_4$ 20 mM glucose). The tube is shaken horizontally (200 rpm) at 37°C for 1 hour. After this 20 μl of cloning mixture is spread on a prewarmed selective LB plate (Luria-Bertani, 10% tryptone, 0.5% yeast extract, 1.0 %NaCl, 1.5% L-agar diluted in water, pH 7), which contains 50 g/ml of ampicillin. The plate is incubated overnight at 37°C. On the next day, ten colonies are chosen from the plate and sequencing PCR is performed.

The reaction mixture (50 μl) for sequencing PCR contains 0.4 pmol of M13 reverse (5'-CAGGAAACAGCTATGAC-3') and M13 forward (5'-GTAAACGACGGCCAG) primers (provided by the kit), 150 μM of each of dATP, dGTP, dTTP, and dCTP (Sigma, USA), 1 x Hot Start Taq PCR buffer (Qiagen, Germany), 1 U Hot Start Taq DNA polymerase (Qiagen, Germany). For the amplification reaction, a small part of bacterial colony is transferred with the help of a sample stick to the PCR reaction tube.

The sequencing PCR is carried out in a GenAmp PCR system 2700 thermal cycler (Applied Biosystems) using the following program: a 15 min denaturation step at 95°C, 30 cycles of 1 min at 94°C, 1 min at 55°C, 1 min at 72°C, and finally a 10 min extension step at 72°C. After the PCR has been performed, the success of amplification is verified by gel electrophoresis using a 2% agarose gel that contains ethidium bromide. After this, the PCR product is purified by removing additional primers, nucleotides, buffer, and polymerase enzyme with a QIAquick PCR purification kit (Qiagen, Germany).

After the purification step the fragment inserted into the vector is sequenced. A 12 μl reaction mixture for the sequencing step contains 100 ng PCR product and 5 pmol of either M13 reverse or M13 forward primer. The sequencing is carried out by a BigDye Terminator Version 3.0 kit and an ABIPRISM 3100 Genetic Analyzer (Applied Biosystems, USA). The sequences are analyzed with the Vector NTI Suite Version 7 program (InforMax, USA).

With the above-described general method *gyrB* sequences were produced for the following bacterial species:

*Moraxella catarrhalis, Fusobacterium necrophorum, Legionella pneumophila, Streptococcus. mitis, Streptococcus oralis,* and *Haemophilus parainfluenzae* (SEQ. ID: NO. 70 to 75).

**Example 3. Design of species-specific probes**

In the design of bacterial species-specific oligonucleotides, i.e. probes, a planning strategy based on alignment was used. The *gyrB* and *parE* genes of target bacterial species were aligned with correspondent genes of a few reference bacteria (closely related bacterial species). For example, the *gyrB* gene of *Streptococcus pneumoniae* was aligned with *gyrB* genes of *Streptococcus pyogenes, Streptococcus mitis, Streptococcus oralis, Mycoplasma hominis, Staphylococcus aureus,* and *Fusobacterium necrophorum. S. oralis* and *S. mitis* are closely related to *S. pneumoniae.* Therefore, oligonucleotides designed for *S. pneumoniae* must not react with these normal flora bacteria. Sequences were obtained from the EMBL public sequence database or they were produced by cloning as described in Example 2.

Alignment of the sequences was performed with the BioEdit program using the ClustalW alignment algorithm. The consensus sequence of the alignments was calculated and the suitably conserved regions were identified manually. These regions refer to sequence fragments that are conserved in the genes of the target bacterial species and that are not found at least entirely from the genes of the reference bacteria. Oligonucleotide sequences with the suitable length (21 - 25 nucleotides) were selected from these areas for comparison analyses. The selected oligonucleotide sequences were compared to the EMBL prokaryotic sequence database using the FastA algorithm program. The oligonucleotide sequences having at least two mismatches when compared to *gyrB*/*parE* sequences of non-target bacteria were chosen for further analyses. Theoretical melting temperature (Tm) was determined for oligonucleotides and the formation of secondary structures was studied. Tm (°C) was calculated with the equation

$$81.5 + 16.6 \log [Na]+0.41(\%GC) - 0.61 (\%for) 500/N,$$

in which Na is the concentration of monovalent cations (50 M is used in calculations), %GC is the proportion of guanine and cytosine, %for is the concentration of formamide (0% is used in calculations) and N is the length of the oligonucleotide. The formation of secondary structures was studied using a program provided by Sigma-Genosys. The program can be used with the help of a web browser at the Internet address http://www.sigma-genosys.co.uk/oligos/frameset.html (calculators/basic calculator). The oligonucleotides that did not form strong secondary structures and whose Tm temperature was at least 45°C were chosen for experimental specificity testing.

Oligonucleotide probes were synthesized and simultaneously modified from the 5' terminus ($NH_2$-modified oligos) (Sigma-Genosys, England). The specificity of the probes was tested in the laboratory with DNA samples isolated from different bacterial species (Table 3) and from patient samples (Table 5) as described in Examples 4 and 5. Of the tested probes, those which functioned best and had the highest specificity were selected. The sequences and specificity of the bacterial species-specific probes are presented in Tables 4A and 4B.

**Table 4A. *GyrB* oligonucleotide sequences.**

| Oligonucleotide/SEQ. ID: NO. | Specificity (*gyrB* gene) | Sequence (5'-3') |
|---|---|---|
| 1/1 | *Streptococcus pneumoniae* | CTCAAAAGAAGGTCTTCACCATC |
| 2/2 | *Streptococcus pneumoniae* | GCCATATTCAAGTTTTTATTGAG |
| 3/3 | *Streptococcus pneumoniae* | AGCCAGATGATTCGATTACTGTT |
| 4/4 | *Streptococcus pneumoniae* | TTGAGACCGTCTTTACAGTCCTT |
| 5/5 | *Streptococcus pyogenes* | GTTTTGCCTCTCATATTAAAGTC |
| 6/6 | *Streptococcus pyogenes* | TCTTTATTGAAGCAGATAATTCC |
| 7/7 | *Streptococcus pyogenes* | CGTTGAAACAGTTTTTACAGTCT |
| 8/8 | *Chlamydia pneumoniae* | GGTCTTCATCATCTAGTCTATGA |
| 9/9 | *Chlamydia pneumoniae* | GGTTGTAGACWACAGCATTGACG |
| 10/10 | *Chlamydia pneumoniae* | AGCCATGGCAGSTTATTGCTCTA |
| 11/11 | *Chlamydia pneumoniae* | GGATTGATGTTSGCATTTTAGAG |
| 12/12 | *Chlamydia pneumoniae* | GGGTATTGTCWTCGTAGATAATG |
| 13/13 | *Chlamydia pneumoniae* | TTATTGCTCTAGGATTGATGTT |
| 14/14 | *Chlamydia pneumoniae* | GCATTTTAGAGSACGGGGGTATT |
| 15/15 | *Mycoplasma pneumoniae* | TCAAACTAACCCTTAAAGACAACT |
| 16/16 | *Mycoplasma pneumoniae* | TGAAACAGTGTTTACGGTACTCC |

(continued)

| Oligonucleotide/SEQ. ID: NO. | Specificity (*gyrB* gene) | Sequence (5'-3') |
|---|---|---|
| 17/17 | *Haemophilus influenzae* | ATGATAATTCTGTATCGGTGCAA |
| 18/18 | *Haemophilus influenzae* | AGCAGAAGTTATTATGACTGTGC |
| 19/19 | *Haemophilus influenzae* | GACGATAACTCTTATAAAGTATC |
| 20/20 | *Haemophilus influenzae* | GATACCGATGACGGTACTGGTTTG |
| 21/21 | *Staphylococcus aureus* | AGTGTGGGAAATTGTCGATAATA |
| 22/22 | *Staphylococcus aureus* | TGAAGTTGTTATTGAAAAAGATAAC |
| 23/23 | *Staphylococcus aureus* | GGATTAAAGTAACGGATAACGGA |
| 24/24 | *Staphylococcus aureus* | CTGTCGAAGTTATTTTAACTGTTT |
| 25/25 | *Staphylococcus aureus* | CGACTTCAGAGAGAGGTTTGCAC |
| 26/26 | *Pseudomonas aeruginosa* | GAAATCAGCATCACCATCCATAC |
| 27/27 | *Pseudomonas aeruginosa* | ATACGGATGAGTCGATCACTGTC |
| 28/28 | *Pseudomonas aeruginosa* | GACGACAACACCTACAAGGTGTC |
| 29/29 | *Pseudomonas aeruginosa* | GACACCGACGATGGCACCGGTCTG |
| 30/30 | *Neisseria gonorrhoeae* | TTCGACAACAACAGCTACAAAAT |
| 31/31 | *Neisseria gonorrhoeae* | ATATGGTGTTTGAAGTATTGGAC |
| 32/32 | *Neisseria gonorrhoeae* | GACAAAATCACGGTAACGATACA |
| 33/33 | *Neisseria gonorrhoeae* | GACAACAACAGCTACAAAATCTC |
| 34/34 | *Escherichia coli* | TATTCGAGGTGGTAGATAACGCT |
| 35/35 | *Escherichia coli* | TTCACGCCGATAACTCTGTCTCT |
| 36/36 | *Escherichia coli* | CGCCGATAACTCTGTCTCTGTAC |
| 37/37 | *Escherichia coli* | GACGATAACTCCTATAAAGTGTC |
| 38/38 | *Escherichia coli* | GACACGGATGACGGCACCGGTCTG |
| 39/39 | *Moraxella catarrhalis* | AGCTGCCGAGGTTATTATGACGG |
| 40/40 | *Moraxella catarrhalis* | GATGATAATTCATACAAAGTATC |
| 41/41 | *Moraxella catarrhalis* | TGTGGATATCCACCCTGAAGAAG |
| 42/42 | *Moraxella catarrhalis* | ATACCGATGATGGTACAGGCTTG |
| 43/43 | *Legionella pneumophila* | GATGATAATTCCTACAAGGTATC |
| 44/44 | *Legionella pneumophila* | AGCAGCCGAAGTCATCATGACAG |
| 45/45 | *Legionella pneumophila* | TGTAGATATTCATAAAGAAGAAG |
| 46/46 | *Legionella pneumophila* | ATACAGATGATGGAACCGGTTTG |
| 47/47 | *Legionella pneumophila* | CAGATGATGGAACCGGTTTGCAT |
| 48/48 | *Fusobacterium necrophorum* | CAACATCAGCCAGGGGATTACAT |
| 49/49 | *Fusobacterium necrophorum* | GGAATTCCAGTAGACATACATCC |
| 50/50 | *Fusobacterium necrophorum* | TGAAAATGATAACTATAAAGTGTC |

**Table 4B. *ParE* oligonucleotide sequences.**

| Oligonucleotide/SEQ. ID: NO. | Specificity (*parE* gene) | Sequence (5'-3') |
|---|---|---|
| 1/51 | *Staphylococcus aureus* | CGGTAACGAAATAGATGTAACAA |
| 2/52 | *Staphylococcus aureus* | AGAAGATAATGGACGTGGTATGC |
| 3/53 | *Staphylococcus aureus* | GTAAACCGACAGTCGAAGTTATC |
| 4/54 | *Staphylococcus aureus* | CAACTGATAAACGGGGATTACATC |
| 5/55 | *Staphylococcus aureus* | GGACAAGGCGGCTATAAAACTTC |
| 6/56 | *Streptococcus pyogenes* | TGGAGATGATATTAAGGTTGTTA |
| 7/57 | *Streptococcus pyogenes* | GTCAGTGTGGCAGATAGCGGACG |
| 8/58 | *Streptococcus pyogenes* | GGATTCCCACCGTTCAAGTTATT |
| 9/59 | *Streptococcus pyogenes* | CAACAGATGCTACGGGATTGCAC |
| 10/60 | *Streptococcus pyogenes* | GGTCAGGGTGGCTACAAAACGTC |
| 11/61 | *Streptococcus pneumoniae* | TGGTGATCGTATTGATGTAACTA |
| 12/62 | *Streptococcus pneumoniae* | CTAACGGTTCAAGACCATGGACG |

(continued)

| Oligonucleotide/SEQ. ID: NO. | Specificity (*parE* gene) | Sequence (5'-3') |
|---|---|---|
| 13/63 | *Streptococcus pneumoniae* | GAATTCCAACTGTTGAGGTTATC |
| 14/64 | *Streptococcus pneumoniae* | CGACCGATGGCGCTGGTCTTCAT |
| 15/65 | *Streptococcus pneumoniae* | GGTCAAGGTGGCTATAAGACATC |
| 16/66 | *Mycoplasma pneumoniae* | TGCTAATACTATTGCCGTTGTTT |
| 17/67 | *Mycoplasma pneumoniae* | ATAACTGTTAGTGACAACGGTCG |
| 18/68 | *Mycoplasma pneumoniae* | AGATTTCTACGATTGACACCGTC |
| 19/69 | *Mycoplasma pneumoniae* | GATAACGATTCTTATAAGATTGC |

**Example 4. Amplification of DNA isolated from patient samples**

DNAs isolated from bacterial culture isolates or clinical patient samples were amplified using the conventional method described below.

DNA is isolated from the sample to be analyzed (a bacterial culture or a clinical patient sample) using the QIAamp DNA Mini kit (Qiagen, Germany). When DNA has been isolated, the desired target strand is amplified using asymmetric polymerase chain reaction (PCR). In the first stage of the amplification, a reaction solution is prepared by mixing together DNA isolated from samples, broad-range gB1F and gB2R primer mixtures (Example1), and other components needed in the amplification.

The PCR reaction mixture contains 32 pmol of gB2R primer mixture, 8 pmol of gB1F primer mixture, 200 μM of each of dATP, dGTP, and dTTP as well as 140 μM dCTP (Sigma, USA), 1 x Hot Start Taq PCR buffer (Qiagen, Germany), in which $MgCl_2$ has been added so that a final concentration is 2.8 mM, 25 nmol Cy5-AP3-dCTP (Amersham Pharmacia Biotech, USA), 7.5% DMSO (Amersham Pharmacia Biotech, USA), 2.5 U Hot Start Taq DNA polymerase (Qiagen, Germany), and 2.5 μl isolated DNA in a total volume of 25 μl.

The PCR is performed using the GenAmp PCR system 2700 thermal cycler (Applied Biosystems, USA). The following PCR program was used: a 15 min denaturation step at 95°C, 40 cycles of 1 min at 95°C, 1 min at 50°C, 1 min at 72°C, and finally a 10 min extension step at 72°C. After the PCR has been performed, the success of amplification is verified by gel electrophoresis using a 2% agarose gel that contains ethidium bromide. After this the Cy5-labeled PCR product is purified by removing additional primers, nucleotides, buffer, and polymerase enzyme with a QIAquick PCR purification kit (Qiagen, Germany).

**Example 5. The design and functioning of sample microarrays**

Oligonucleotide probes aminated at the 5' terminus (designed according to Example 3) were dissolved in 400 mM sodium carbonate buffer (pH 9.0) to a final concentration of 50 μM. The probes were covalently attached onto aminosilane coated microscope slides (Genorama, Asper Biotech Ltd., Estonia). The transfer of the probes to the glass slides was performed with a robot developed for this purpose (OmniGrid, GeneMachines, USA) and pins (Telechem SMP3, USA). The average size of the printed probe area was 120 μm. Moreover, positive control primers aminated at the 5' terminus were printed onto the glass slides. After printing the microarray slides were kept in ammonia vapor for 1 hour in order to attach the probes to the slides. After the ammonia treatment they were washed three times with sterile water and dried.

Next, a Cy5-labeled target strand (manufactured according to Example 4) was hybridized to the microscope slide where the probes had been attached. The hybridization reaction mixture contained about 200 - 300 ng target strand, 20 x SSC (1 μl 20xSSC contains 175.3 g NaCl and 88.2 g sodium citrate, pH is adjusted to 7.0 with HCl; the final concentration is 3.4x), 20% sodium dodecyl sulphate (SDS) (a final concentration of 0.3 %), and sterile water so that the volume of the reaction mixture was 37 μl. First the mixture was denatured at 95°C for 3 min. After this the tubes were immediately transferred onto ice. After the mixture had cooled down, it was pipetted onto the cover slip that was placed against the glass slide on which the probes had been attached. The microarray slide was placed inside the hybridization chamber (Arraylt, TeleChem International, USA) and the chamber was shut tight. Finally, the hybridization chamber was immersed in a water bath. The microarray slides were hybridized at 57°C for 12 - 16 hours.

After hybridization the microarray slides were washed in three different washing solutions in order to remove non-hybridized DNA. The washing steps were carried out as follows: in 0.1% SDS solution for 5 min at 57°C, in 0.1% SDS, 0.5xSSC washing solution for 5 min at room temperature, and in 0.06xSSC for 5 min at room temperature.

After the glass slides had dried, they were analyzed with a microarray scanner (Agilent DNA Microarray Scanner, Agilent, USA). If the Cy5-labeled target strand had bound to one or several probes, these spots emitted a fluorescent signal. Furthermore, positive control probes also gave a fluorescent signal.

An example of a hybridization result is presented in Figure 2. *A gyrB* DNA fragment (an asymmetric Cy5-labeled PCR product) isolated from a culture isolate of *Staphylococcus aureus* was used as the target strand. This example slide included four *gyrB* oligonucleotides that are specific for *Staphylococcus aureus* (Table 4B: oligonucleotides 22 - 25). All of them bound specifically the target strand? of *Staphylococcus aureus* and gave a fluorescent signal. Furthermore, the five positive control oligonucleotides gave a fluorescent signal.

**Example 6. Analysis of patient samples**

DNA from clinical samples obtained from patients suffering from respiratory tract infections was isolated and amplified using the method described in Example 4. The samples were tested using the microarray slide (described in Example 5) on which the probes and positive control oligonucleotides (listed in Table 4A and 4B) were attached. The same samples were also analyzed with the method based on broad-range PCR, as earlier described in Nikkari *et al.* (Emerging Infectious Diseases, vol 8, no. 2, 2002, s.188 - 194) and Kotilainen *et al.* (Journal of Clinical Microbiology, vol 36, no. 8, 1998, s. 2205 - 2209).

Broad-range primers originating from the 16S rRNA gene area were used in the broad-range bacterial PCR method. They were named as fD1 mod and 16S1RR-B primers in earlier publications. The PCR product was cloned and sequenced. The DNA sequence was compared to public sequence databases (GenBank) and the bacterial species was thus identified. The results are presented in Table 5.

As can be seen from Table 5, the results obtained with the method of the invention are entirely identical when compared to the conventional broad-range PCR method. The method defined by the present invention is substantially faster to perform, as results are available in approximately one day. On the other hand, it takes about one week on average to perform broad-range bacterial PCR including the time-consuming cloning and sequencing steps. Compared to culture testing, both the method of the present invention and broad-range bacterial PCR turned out to be as sensitive as cultivation and even more sensitive in some cases.

**Table 5. Comparison between the different methods using patient samples**

| Clinical picture | Sample | Culture result | Broad-range PCR | The method of invention |
|---|---|---|---|---|
| Tonsillitis | Tissue | no information | F.N. | F.N. |
| Pneumonia 1) | Sputum | not cultured | no information | S.P. |
| Otitis media | Pus | *H.I.* | *H.I.* and *M.C.* | *H.I.* and *M.C.* |
| Otitis media | Pus | *S.P.* | *S.P.* | *S.P.* |
| Otitis media | Pus | negative | *H.I.* | *H.I* |
| Otitis media | Pus | *H.I.* | *H.I.* | *H.I.* |
| Otitis media | Pus | *H.I., S.P.* | *H. I., M.C.* and *S.P* | *H.I*, *M.C.* and *S.P.* |
| Maxillary sinusitis | Pus | *H.I.* | *H.I.* | *H.I.* |
| Maxillary sinusitis | Pus | negative | *H.I.* | *H.I.* |

*F.N. = Fusobacterium necrophorum*
*S.P. = Streptococcus pneumoniae*
H.I. *= Haemophilus influenzae*
*M.C. = Moraxella catarrhalis*
1) Bacterial culture and broad-range PCR were not performed from the sample isolated from the patient suffering from pneumonia, only the method of the present invention was performed. With this method *Streptococcus pneumoniae* was found from the sample. This result is compatible with the clinical picture, as this bacterial species is one of the most common causative agents of pneumonia.

**Example 7. Comparison between the method of the present invention and aknown method that is based on specific oligonucleotides**

The identification of multiple bacterial pathogens is disturbed by bacteria of normal flora. The presence of these bacteria is necessary for the health of a human organism, and it has been estimated that hundreds of different bacteria belong to human normal flora. Thus the bacteria of normal flora can in many cases disturb bacterial diagnostics.

The specificity of the method of the present invention, in which specific *gyrB*/*parE* oligonucleotide probes are used, was studied with respect to normal flora. Simultaneously, the method was compared to the multiplex PCR method where a mixture of broad-range and species-specific primers is used for amplification.

DNA was isolated from culture isolates of the normal flora bacteria *Streptococcus mitis, Streptococcus oralis, Moraxella caviae,* and *Moraxella cuniculi.* After this, isolated DNA was amplified using the method described in Example 4 and tested using the microarray slide (described in Example 5), on which specific probes (presented in Table 4A and 4B) had been attached. For comparison, specific PCR primers described in the publication of Hendolin *et al.* (Journal of Clinical Microbiology, 35; 11, 1997) were synthesized and a variant of the multiplex PCR was performed by using the method described in this publication. In this variant of the conventional multiplex PCR method the broad-range primer originating from the conserved gene region of 16S rRNA is used as one PCR primer and the mixture of primers that consists of four different species-specific primers is used as another PCR primer. Bacterial species to be diagnosed with this primer pair were *Alloiococcus otitidis, Haemophilus influenzae, Moraxella catarrhalis,* and *Streptococcus pneumoniae.* Species-specific primers were designed so that the amplified PCR product differs in length depending on from which bacterial species it has been isolated. The results are presented in Table 6.

The results demonstrate that with the method of the present invention, in which specific oligonucleotide probes are used, only the desired bacterial species are specifically identified. Instead, specific primers defined by the known method and the multiplex PCR method cannot distinguish the desired bacteria from the studied bacteria of normal flora, and thus are not sufficiently specific (Figure 3).

**Table 6. Comparison between the method of the present invention and the multiplex PCR method**

| **Bacterial species** | ***) Probes on which they bound (specificity)** | **Multiplex PCR result** |
|---|---|---|
| *Streptococcus pneumoniae*<br>*Streptococcus pyogenes* | 4B: 10 - 14 (*Strep.pneu*)<br>4A: 1 - 4 (*Strep.pneu*)<br>4B: 5 - 9 (*Strep.pyo*)<br>4A. 5 - 7 (*Strep. pyo*) | *Strep. pneu* was amplified with specific primers<br>*Strep.pneu* was not amplified with specific primers |
| *Streptococcus oralis* | No binding | *Strep.pneu* was amplified with specific primers |
| *Streptococcus mitis* | No binding | *Strep.pneu* was amplified with specific primers |
| *Moraxella catarrhalis* | 4A: 39 - 42 (*Morax. catarr)* | *Morax.cat.* was amplified with specific primers |
| *Moraxella cuniculi* | No binding | *Morax.cat.* was amplified with specific primers |
| *Moraxella caviae* | No binding | *Morax.cat.* was amplified with specific primers |
| *Neisseria gonorrhoae* | 4A: 30 - 33 (*Neisseria gon.*) | *Morax.cat.* was not amplified with specific primers |
| *) 4A means Table 4A and 4B Table 4B. Numbers (e.g., 10 to 14) refer to the numbers of oligonucleotides in the previously mentioned Tables. Specificity indicates for which bacterial species the oligonucleotide probes have been designed. | | |

**Claims**

1. A diagnostic method for detecting and identifying bacterial species causing infections from a clinical sample, **characterized by**

   a) amplifying DNA isolated from said clinical sample using a mixture of DNA primers that comprises sequences which hybridize with the sequences that originate from conserved regions of genes encoding topoisomerases, especially *gyrB*/*parE,* of bacterial species causing said infections, said sequences comprising SEQ ID NOS:

76 and 77 or with complementary sequences thereof,
b) contacting the amplified DNA with a desired combination of oligonucleotide probe sequences that hybridize under normal hybridization conditions with hyper-variable regions situated near said conserved regions of genes encoding topoisomerases, especially *gyrB*/*parE*, of bacterial species causing said infections, said sequences being bacterial species-specific under said hybridization conditions, and
c) detecting the formation of a possible hybridization complex.

**2.** The diagnostic method according to claim 1, **characterized in that** said infections causing bacterial species are bacterial species that cause respiratory tract infections.

**3.** The diagnostic method according to claim 1 or 2, **characterized in that** said hyper-variable region is the hyper-variable region of the gene encoding the *gyrB* and/or *parE* protein of a bacterial species selected from *Streptococcus pneumoniae, Streptococcus pyogenes, Chlamydia pneumoniae, Mycoplasma pneumoniae, Haemophilus influenzae, Staphylococcus aureus, Pseudomonas aeruginosa, Neisseria gonorrhoeae, Escherichia coli, Moraxella catarrhalis, Legionella pneumophila,* and *Fusobacterium necrophorum.*

**4.** The diagnostic method according to any one of claims 1 to 3, **characterized in that** the length of oligonucleotide probe sequences used in step b) is 15 - 30, more preferably 20 - 30, and most preferably 21 - 25 nucleic acids.

**5.** The diagnostic method according to any one of claims 1 to 4, **characterized in that** said combination of oligonucleotide probe sequences comprises all or a portion of the SEQ ID NOS: 1 to 69, and/or complementary sequences thereof.

**6.** The diagnostic method according to claim 5, **characterized in that** said combination of oligonucleotide probe sequences comprises all the SEQ ID NOS: 1 to 69.

**7.** The diagnostic method according to any one of claims 1 to 6, **characterized in that** said combination of oligonucleotide probe sequences is attached onto a solid support.

**8.** The diagnostic method according to claim 1, **characterized in that** the DNA isolated from the clinical sample in step a) is amplified using the polymerase chain reaction (PCR) and that the DNA amplified in step b) is contacted with bacterial species-specific oligonucleotide probes attached onto a solid support.

**9.** The diagnostic method according to claim 7 or 8, **characterized in that** said solid support is treated glass.

**10.** The diagnostic method according to claim 1, **characterized in that** suitably labeled nucleotides are used in the amplification of DNA isolated from a clinical sample in step a) to generate a detectable target strand.

**11.** The diagnostic method according to claim 10, **characterized in that** the amplified and optionally labeled target DNA in step b) is contacted with a solid support, on which all bacterial species-specific oligonucleotide probes SEQ ID NOS: 1 to 69 and/or complementary sequences thereof have been attached.

**12.** The diagnostic method according to claim 10, **characterized in that** the amplified and optionally labeled target DNA in step b) is contacted with a solid support on which specific oligonucleotide probe sequences detecting one specified bacterial species or a few specified bacterial species causing infections have been attached, said sequences being selected from SEQ ID NOS: 1 to 4 and 61 to 65 of *Streptococcus pneumoniae,* 5 to 7 and 56 to 60 of *Streptococcus pyogenes,* 8 to 14 of *Chlamydia pneumoniae*, 15 to 16 and 66 to 69 of *Mycoplasma pneumoniae,* 17 to 20 of *Haemophilus influenzae,* 21 to 25 and 51 to 55 of *Staphylococcus aureus* , 26 to 29 of *Pseudomonas aeruginosa,* 30 to 33 of *Neisseria gonorrhoeae,* 34 to 38 of *Escherichia coli,* 39 to 42 of *Moraxella catarrhalis,* 43 to 47 of *Legionella pneumophila* and 48 to 50 of *Fusobacterium necrophorum,* and/or complementary sequences thereof.

**13.** The diagnostic method according to any one of claims 1-12, **characterized in that** the microarray technology is used in step c).

**14.** A DNA primer mixture, **characterized by** comprising sequences that hybridize with sequences of the conserved regions of genes encoding topoisomerases, especially the *gyrB* and/or *parE* proteins, of bacterial species that cause infections, especially bacterial species that cause respiratory tract infections, said mixture comprising SEQ ID NOS:

76 and 77 and/or reversed or complementary sequences thereof.

**15.** An oligonucleotide sequence useful in the diagnosis of infection causing bacterial species, **characterized in that** it hybridizes under normal hybridization conditions with a sequence of a hyper-variable region that is bacterial species-specific and is situated near the conserved regions of genes encoding topoisomerases, especially the *gyrB* and/or *parE* proteins, said oligonucleotide sequence being one of the SEQ ID NOS: 1 to 69 and/or complementary sequences thereof.

**16.** The combination of oligonucleotide probe sequences useful in the diagnosis of infection causing bacterial species, **characterized by** comprising any combination of the SEQ ID NOS: 1 to 69 and/or complementary sequences thereof.

**17.** The combination of oligonucleotide probes according to claim 16, **characterized by** comprising all of the SEQ. ID. NO: 1 to 69.

**18.** The use of the combination of oligonucleotide probes according to claim 16 or 17 for the detection, identification, or classification of infection causing bacterial species.

**19.** A diagnostic kit for use in the diagnosis of infection-causing bacteria, especially those causing respiratory tract infections, **characterized by** comprising

a) a DNA primer mixture comprising sequences that hybridize with sequences of the conserved regions of genes encoding topoisomerases, especially the gyrB and/or parE proteins, of bacterial species that cause infections, especially bacterial species that cause respiratory tract infections, said mixture comprising SEQ ID NOS: 76 and 77 and/or complementary sequences thereof of the invention as defined above,
b) a combination of bacterial species-specific oligonucleotide probe sequences, optionally attached on a solid support, comprising any combination of the SEQ ID NOS: 1 to 69 and/or reverse or complementary sequences thereof.
c) positive and optionally negative control probe sequences, and optionally
d) reagents required in the amplification, hybridisation, purification washing, and/or detection steps.

## Patentansprüche

**1.** Diagnoseverfahren zum Nachweis und zur Identifizierung von Bakterienspezies, die Infektionen verursachen, aus einer klinischen Probe, **gekennzeichnet durch**

a) Amplifizieren von aus der klinischen Probe isolierter DNA unter Verwendung eines Gemisches von DNA-Primern, das Sequenzen umfasst, die mit den Sequenzen, die aus konservierten Regionen von Topoisomerasen codierenden Genen, insbesondere *gyrB*/*parE,* von Bakterienspezies, die die Infektionen verursachen, stammen, wobei die Sequenzen die SEQ ID NOS: 76 und 77 umfassen, oder mit komplementären Sequenzen davon hybridisieren,
b) Kontaktieren der amplifizierten DNA mit einer gewünschten Kombination von Oligonucleotid-Sondensequenzen, die unter normalen Hybridisierungsbedingungen mit hypervariablen Regionen hybridisieren, die in der Nähe der konservierten Regionen von Topoisomerasen codierenden Genen, insbesondere *gyrB*/*parE,* von Bakterienspezies, die die Infektionen verursachen, liegen, wobei die Sequenzen unter den Hybridisierungsbedingungen Bakterienspezies-spezifisch sind, und
c) Nachweisen der Bildung eines möglichen Hybridisierungskomplexes.

**2.** Diagnoseverfahren nach Anspruch 1, **dadurch gekennzeichnet, dass** die Infektionen verursachenden Bakterienspezies Bakterienspezies sind, die Atemwegsinfektionen hervorrufen.

**3.** Diagnoseverfahren nach Anspruch 1 oder 2, **dadurch gekennzeichnet, dass** die hypervariable Region in der hypervariablen Region des Gens liegt, das das *gyrB*-und/oder parE-Protein einer Bakterienspezies codiert, die aus *Streptococcus pneumoniae, Streptococcus pyogenes, Chlamydia pneumoniae, Mycoplasma pneumoniae, Haemophilus influenzae, Staphylococcus aureus, Pseudomonas aeruginosa, Neisseria gonorrheae, Escherichia coli, Moraxella catarrhalis, Legionella pneumophila* und *Fusobacterium necrophorum* ausgewählt ist.

**4.** Diagnoseverfahren nach einem der Ansprüche 1 bis 3, **dadurch gekennzeichnet, dass** die Länge von Oligonucleotid-Sondensequenzen, die in Schritt b) verwendet werden, 15-30, stärker bevorzugt 20-30 und am stärksten bevorzugt 21-25 Nucleinsäuren beträgt.

**5.** Diagnoseverfahren nach einem der Ansprüche 1-4, **dadurch gekennzeichnet, dass** die Kombination von Oligonucleotid-Sondensequenzen alle oder einen Teil der SEQ ID NOS: 1-69 und/oder Komplementärsequenzen davon umfasst.

**6.** Diagnoseverfahren nach Anspruch 5, **dadurch gekennzeichnet, dass** die Kombination von Oligonucleotid-Sondensequenzen alle SEQ ID NOS: 1-69 umfasst.

**7.** Diagnoseverfahren nach einem der Ansprüche 1 bis 6, **dadurch gekennzeichnet, dass** die Kombination von Oligonucleotid-Sondensequenzen an einen festen Träger gebunden ist.

**8.** Diagnoseverfahren nach Anspruch 1, **dadurch gekennzeichnet, dass** die in Schritt a) aus der klinischen Probe isolierte DNA unter Verwendung der Polymerasekettenreaktion (PCR) amplifiziert wird und dass die in Schritt b) amplifizierte DNA mit Bakterienspezies-spezifischen Oligonucleotid-Sonden, die an einen festen Träger gebunden sind, kontaktiert wird.

**9.** Diagnoseverfahren nach Anspruch 7 oder 8, **dadurch gekennzeichnet, dass** der feste Träger behandeltes Glas ist.

**10.** Diagnoseverfahren nach Anspruch 1, **dadurch gekennzeichnet, dass** in zweckmäßiger Weise markierte Nucleotide bei der Amplifikation von DNA, die aus einer klinischen Probe in Schritt a) isoliert wurde, verwendet werden, um einen nachweisbaren Zielstrang zu erzeugen.

**11.** Diagnoseverfahren nach Anspruch 10, **dadurch gekennzeichnet, dass** die in Schritt b) amplifizierte und gegebenenfalls markierte Ziel-DNA mit einem festen Träger kontaktiert wird, an den sämtliche Bakterienspezies-spezifischen Oligonucleotidsonden SEQ ID NOS: 1-69 und/oder komplementäre Sequenzen davon gebunden wurden.

**12.** Diagnoseverfahren nach Anspruch 10, **dadurch gekennzeichnet, dass** die in Schritt b) amplifizierte und gegebenenfalls markierte Ziel-DNA mit einem festen Träger kontaktiert wird, an den spezifische Oligonucleotidsonden-Sequenzen gebunden wurden, die eine festgelegte Bakterienspezies oder einige festgelegte Bakterienspezies, die Infektionen verursachen, nachweisen, wobei die Sequenzen ausgewählt sind aus SEQ ID NOS: 1-4 und 61-65 von *Streptococcus pneumoniae,* 5 bis 7 und 56 bis 60 von *Streptococcus pyogenes,* 8 bis 14 von *Chlamydia pneumoriae, 15* bis 16 und 66 bis 69 von *Mycoplasma pneumoniae,* 17 bis 20 von *Haemophilus influenzae,* 21 bis 25 und 51 bis 55 von *Staphylococcus aureus,* 26 bis 29 von *Pseudomonas aeruginosa,* 30 bis 33 von *Neisseria gonorrheae,* 34 bis 38 von *Escherichia coli,* 39 bis 42 von *Moraxella catarrhalis,* 43 bis 47 von *Legionella pneumophila* und 48 bis 50 von *Fusobacterium necrophorum* und/oder komplementären Sequenzen davon.

**13.** Diagnoseverfahren nach einem der Ansprüche 1-12, **dadurch gekennzeichnet, dass** in Schritt c) die Microarray-Technik verwendet wird.

**14.** DNA-Primergemisch, **dadurch gekennzeichnet, dass** es Sequenzen umfasst, die mit Sequenzen der konservierten Regionen von Genen hybridisieren, die Topoisomerasen, insbesondere die *gyrB*- und/oder *parE*-Proteine, von Bakterienspezies, codieren, die Infektionen verursachen, insbesondere von Bakterienspezies, die Atemwegsinfektionen verursachen, wobei das Gemisch die SEQ ID NOS: 76 und 77 und/oder reverse oder komplementäre Sequenzen davon umfasst.

**15.** Oligonucleotid-Sequenz, die bei der Diagnose einer infektionsverursachenden Bakterienspezies geeignet ist, **dadurch gekennzeichnet, dass** sie unter normalen Hybridisierungsbedingungen mit einer Sequenz einer hypervariablen Region hybridisiert, die Bakterienspezies-spezifisch ist und in der Nähe der konservierten Regionen von Genen liegt, die Topoisomerasen, insbesondere die *gyrB*- und/oder *parE*-Proteine, codieren, wobei die Oligonucleotid-Sequenz eine der SEQ ID NOS: 1-69 und/oder komplementäre Sequenzen davon ist.

**16.** Kombination von Oligonucleotid-Sondensequenzen, die bei der Diagnose von infektionsverursachenden Bakterienspezies geeignet sind, **dadurch gekennzeichnet, dass** sie eine beliebige Kombination der SEQ ID NOS: 1-69 und/oder komplementäre Sequenzen davon umfassen.

**17.** Kombination von Oligonucleotidsonden nach Anspruch 16, **dadurch gekennzeichnet, dass** sie alle von SEQ ID NO: 1-69 umfassen.

18. Verwendung der Kombination von Oligonucleotidsonden nach Anspruch 16 oder 17 zum Nachweis, zur Identifikation oder Klassifizierung von infektionsverursachenden Bakterienspezies.

19. Diagnostischer Testsatz zur Verwendung bei der Diagnose von infektionsverursachenden Bakterien, insbesondere denjenigen, die Atemwegsinfektionen verursachen, **dadurch gekennzeichnet, dass** es umfasst

a) ein DNA-Primer-Gemisch, umfassend Sequenzen, die mit Sequenzen der konservierten Regionen von Genen hybridisieren, die Topoisomerasen, insbesondere die *gyrB*- und/oder *parE*-Proteine, von bakteriellen Spezies, die Infektionen verursachen, codieren, insbesondere von bakteriellen Spezies, die Atemwegsinfektionen verursachen, wobei das Gemisch die SEQ ID NOS: 76 und 77 und/oder erfindungsgemäße komplementäre Sequenzen davon, wie vorstehend definiert, umfasst,
b) eine Kombination von Bakterienspezies-spezifischen Oligonucleotid-Sondensequenzen, die gegebenenfalls an einen festen Träger gebunden sind, umfassend eine beliebige Kombination der SEQ ID NOS: 1-69 und/oder reverse oder komplementäre Sequenzen davon,
c) positive und gegebenenfalls negative Kontrollsondensequenzen, und gegebenenfalls
d) Reagenzien, die bei dem Amplifikations-, Hybridisierungs-, Reinigungs-, Wasch- und/oder Nachweisschritt erforderlich sind.

## Revendications

1. Procédé de diagnostic pour détecter et identifier des espèces bactériennes provoquant des infections à partir d’un échantillon clinique, **caractérisé par**

a) l’amplification d’un ADN isolé à partir dudit échantillon clinique en utilisant un mélange d’amorces ADN qui comprend des séquences qui s’hybrident aux séquences provenant de régions conservées de gènes codant pour des topoisomérases, particulièrement *gyrB*/*parE*, d’espèces bactériennes provoquant lesdites infections, lesdites séquences comprenant les SEQ ID N° : 76 et 77, ou les séquences complémentaires de celles-ci,
b) la mise en contact de l’ADN amplifié avec une combinaison souhaitée de séquences de sonde oligonucléotidique qui s’hybrident, dans des conditions d’hybridation normales, à des régions hypervariables situées à proximité desdites régions conservées de gènes codant pour des topoisomérases, particulièrement *gyrB*/*parE,* d’espèces bactériennes provoquant lesdites infections, lesdites séquences étant spécifiques à une espèce bactérienne dans lesdites conditions d’hybridation, et
c) la détection de la formation d’un éventuel complexe d’hybridation.

2. Procédé de diagnostic selon la revendication 1, **caractérisé en ce que** lesdites espèces bactériennes provoquant des infections sont des espèces bactériennes qui provoquent des infections des voies respiratoires.

3. Procédé de diagnostic selon la revendication 1 ou 2, **caractérisé en ce que** ladite région hyper-variable est la région hypervariable du gène codant pour la protéine *gyrB* et/ou *parE* d’une espèce bactérienne sélectionnée parmi *Streptococcus pneumoniae, Streptococcus pyogenes, Chlamydia pneumoniae, Mycoplasma pneumoniae, Haemophilus influenzae, Staphylococcus aureus, Pseudomonas aeruginosa, Neisseria gonorrhoeae, Escherichia coli, Moraxella catarrhalis, Legionella pneumophila,* et *Fusobacterium necrophorum.*

4. Procédé de diagnostic selon l’une quelconque des revendications 1 à 3, **caractérisé en ce que** la longueur des séquences de sonde oligonucléotidique utilisées dans l’étape b) est de 15-30, de manière plus préférée de 20-30, et de la manière la plus préférée entre toutes de 21-25 acides nucléiques.

5. Procédé de diagnostic selon l’une quelconque des revendications 1 à 4, **caractérisé en ce que** ladite combinaison de séquences de sonde oligonucléotidique comprend l’intégralité ou une partie des SEQ ID N° : 1 à 69, et/ou des séquences complémentaires de celles-ci.

6. Procédé de diagnostic selon la revendication 5, **caractérisé en ce que** la dite combinaison de séquences de sonde oligonucléotidique comprend l’intégralité des SEQ ID N° : 1 à 69.

7. Procédé de diagnostic selon l’une quelconque des revendications 1 à 6, **caractérisé en ce que** ladite combinaison de séquences de sonde oligonucléotidique est attachée à un support solide.

8. Procédé de diagnostic selon la revendication 1, **caractérisé en ce que** l'ADN isolé à partir de l'échantillon clinique de l'étape a) est amplifié en utilisant la réaction en chaîne par polymérase (PCR) et **en ce que** l'ADN amplifié à l'étape b) est mis en contact avec des sondes oligonucléotidiques spécifiques d'espèces bactériennes attachées à un support solide.

9. Procédé de diagnostic selon la revendication 7 ou 8, **caractérisé en ce que** ledit support solide est du verre traité.

10. Procédé de diagnostic selon la revendication 1, **caractérisé en ce que** des nucléotides marqués de manière appropriée sont utilisés pour l'amplification de l'ADN isolé à partir d'un échantillon clinique de l'étape a) afin de générer un brin cible détectable.

11. Procédé de diagnostic selon la revendication 10, **caractérisé en ce que** l'ADN cible amplifié et facultativement marqué à l'étape b) est mis en contact avec un support solide, sur lequel toutes les sondes oligonucléotidiques spécifiques d'espèces bactériennes de SEQ ID N° : 1 à 69, et/ou des séquences complémentaires de celles-ci, ont été attachées.

12. Procédé de diagnostic selon la revendication 10, **caractérisé en ce que** l'ADN cible amplifié et facultativement marqué à l'étape b) est mis en contact avec un support solide sur lequel des séquences de sonde oligonucléotidique spécifiques, détectant une espèce bactérienne spécifiée ou quelques espèces bactériennes spécifiées provoquant des infections, ont été attachées, lesdites séquences étant sélectionnées parmi SED ID N° : 1 à 4 et 61 à 65 de *Streptococcus pneumoniae,* 5 à 7 et 56 à 60 de *Streptococcus pyogenes,* 8 à 14 de *Chlamydia pneumoniae,* 15 à 16 et 66 à 69 de *Mycoplasma pneumoniae,* 17 à 20 de *Haemophilus influenzae,* 21 à 25 et 51 à 55 de *Staphylococcus_aureus,* 26 à 29 de *Pseudomonas aeruginosa,* 30 à 33 de *Neisseria gonorrhoaea,* 34 à 38 de *Escherichia coli*, 39 à 42 de *Moraxella catarrhalis,* 43 à 47 de *Legionella pneumophila* et 48 à 50 de *Fusobacterium necrophorum,* et/ou des séquences complémentaires de celles-ci.

13. Procédé de diagnostic selon l'une quelconque des revendications 1 à 12, **caractérisé en ce que** la technologie de puces à ADN est utilisée pour l'étape c).

14. Mélange d'amorces ADN, **caractérisé en ce qu'**il comprend des séquences qui s'hybrident à des séquences des régions conservées de gènes codant pour des topoisomérases, particulièrement les protéines *gyrB* et/ou *parE,* d'espèces bactériennes provoquant des infections, particulièrement des espèces bactériennes qui provoquent des infections des voies respiratoires, ledit mélange comprenant les SED ID N° : 76 et 77 et/ ou des séquences inverses ou complémentaires de celles-ci.

15. Séquence oligonucléotidique utile dans le diagnostic d'une infection provoquée par une espèce bactérienne, **caractérisée en ce qu'**elle s'hybride, dans des conditions d'hybridation normales, à une séquence d'une région hypervariable qui est spécifique à une espèce bactérienne et qui est située à proximité des régions conservées de gènes codant pour des topoisomérases, particulièrement les protéines *gyrB* et/ ou *parE,* ladite séquence oligonucléotidique étant une des SED ID N° : 1 à 69 et/ou des séquences complémentaires de celles-ci.

16. Combinaison de séquences de sonde oligonucléotidique utiles dans le diagnostic d'une infection provoquée par une espèce bactérienne, **caractérisée en ce qu'**elle comprend une quelconque combinaison des SEQ ID N° 1 à 69 et/ou des séquences complémentaires de celles-ci.

17. Combinaison de séquences de sonde oligonucléotidique selon la revendication 16, **caractérisée en ce qu'**elle comprend l'intégralité des SEQ ID N° : 1 à 69.

18. Utilisation de la combinaison de sondes oligonucléotidiques selon la revendication 16 ou 17 pour la détection, l'identification, ou la classification d'espèces bactériennes provoquant une infection.

19. Kit de diagnostic destiné à une utilisation dans le diagnostic de bactéries provoquant une infection, particulièrement celles provoquant des infections des voies respiratoires, **caractérisé en ce qu'**il comprend

a) un mélange d'amorces ADN comprenant des séquences qui s'hybrident aux séquences des régions conservées de gènes codant pour des topoisomérases, particulièrement les protéines *gyrB* et/ou *parE,* d'espèces bactériennes qui provoquent des infections, particulièrement des espèces bactériennes qui provoquent des infections des voies respiratoires, ledit mélange comprenant les SED ID N° : 76 et 77 et/ou des séquences complémentaires de celles-ci de l'invention tel que défini ci-dessus,
b) une combinaison de séquences de sonde oligonucléotidique spécifiques d'espèces bactériennes, facultativement attachées à un support solide, comprenant une quelconque combinaison des SEQ ID N° : 1 à 69 et/ou des séquences inverses ou complémentaires de celles-ci.
c) des séquences de sonde de contrôle positif et facultativement de contrôle négatif, et facultativement
d) des réactifs qui sont nécessaires dans les étapes d'amplification, d'hybridation, de purification, de lavage, et/ou de détection.

# Fig. 1.

**A. The *gyrB* amplification product of *Haemophilus influenzae* and the location of the probes**

21
5'-cgtcctggtatgtatatcggcgataccgatgacggtactggtttgcacc
atatggtatttgaagtggtggataatgccattgatgaagccctcgctggcc
18
attgttccgatattatcgtgacaattcacgatgataattctgtatcggtgcaa
gatgatgggggcgggattcctgtggatattcatcctgaagaaggcgtttct
19
gcagcagaagttattatgactgtgcttcatcgcaggcggtaaatttgacga
20
aactcttataaagtatcgggcggtttacacggcgtggg-3'

**B. The *gyrB* amplification product of *Moraxella catarrhalis* and the location of the probes**

43
5'-cgaccagggatgtatattggtgataccgatgatggtacaggcttgca
ccatatggtgtttgaggtggtggatatgccattgatgaggcattggcaggtc
actgtgatgagattaatattatcgtccatgacgatgaatctgtttcggtgatg
42
gagtatgggcgtggtattcctgtggatatccaccctgaagaaggtgtatc
40
agctgccgaggttattatgacggtgcttcatgcaggcggtaaatttgatgat
41
aattcatacaaagtatctgggggcctgcacggcgtagg-3'

Fig. 2

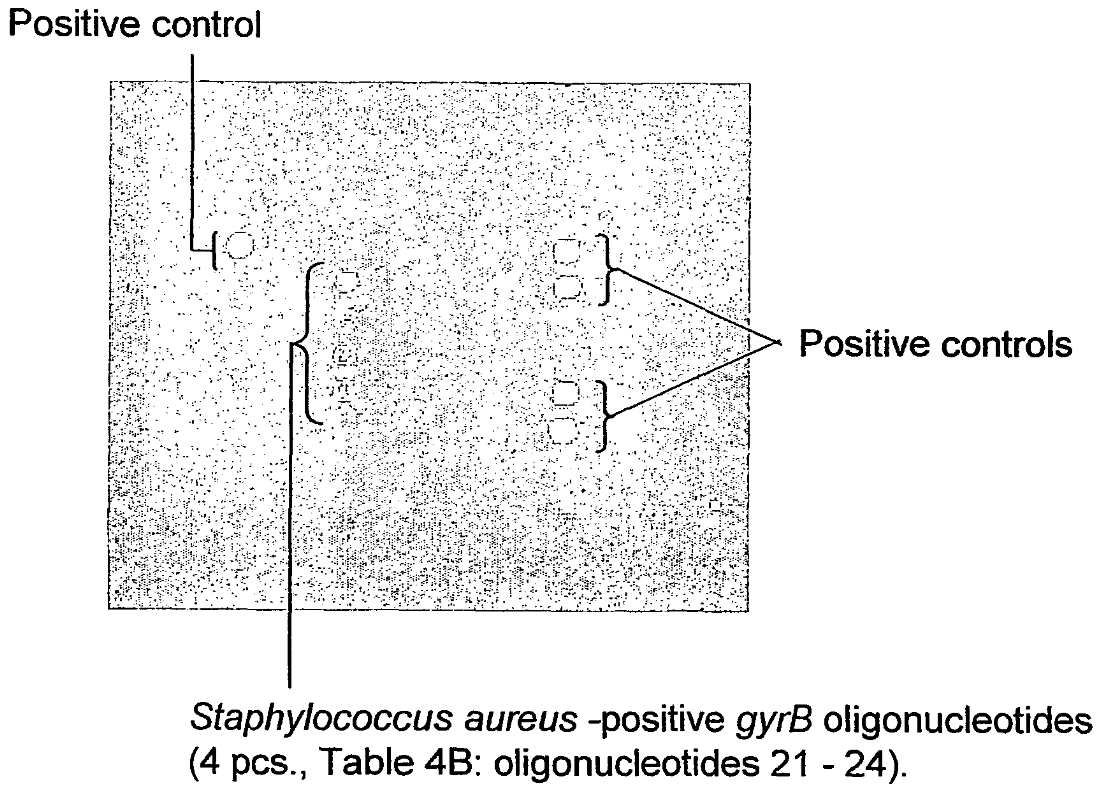
Positive control
Positive controls
*Staphylococcus aureus* -positive *gyrB* oligonucleotides
(4 pcs., Table 4B: oligonucleotides 21 - 24).

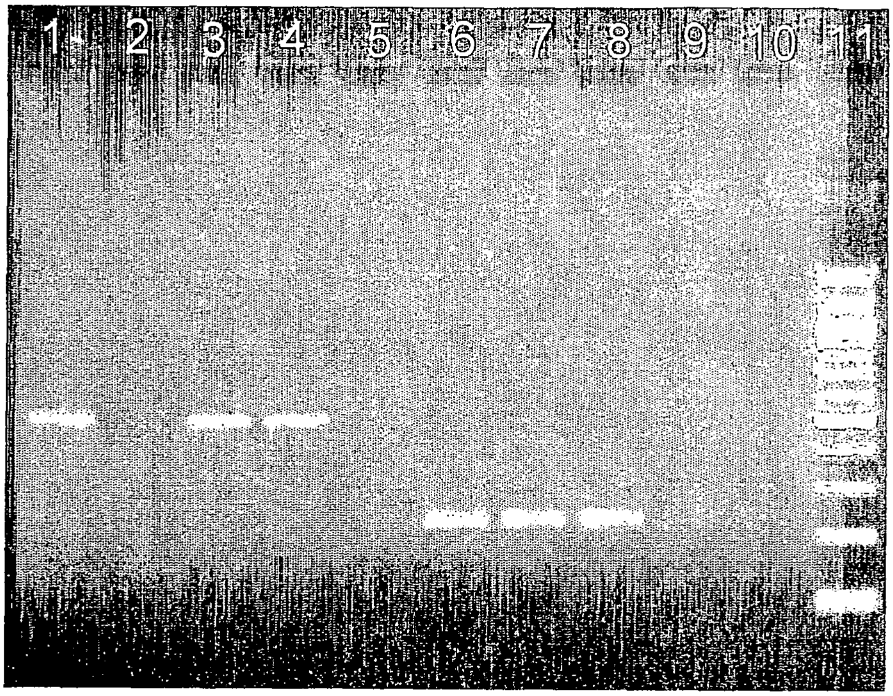


Fig. 3

1. *Streptococcus pneumoniae*
2. *Streptococcus pyogenes*
3. *Streptococcus oralis*
4. *Streptococcus mitis*
5. Negative control
6. *Moraxella catarrhalis*
7. *Moraxella cuniculi*
8. *Moraxella caviae*
9. *Neisseria gonorrhoeae*
10. Negative control
11. Size marker

SEQUENCE LISTING

<110> MoBiDiag Oy

<120> Nucleic acid probes, broad-range primers, and methods in which they are used

<130> 2022264FI

<160> 77

<170> PatentIn version 3.1

<210> 1
<211> 23
<212> DNA
<213> Streptococcus pneumoniae

<400> 1
ctcaaaagaa ggtcttcacc atc 23

<210> 2
<211> 23
<212> DNA
<213> Streptococcus pneumoniae

<400> 2
gccatattca agtttttatt gag 23

<210> 3
<211> 23
<212> DNA
<213> Streptococcus pneumoniae

<400> 3
agccagatga ttcgattact gtt 23

<210> 4
<211> 23
<212> DNA
<213> Streptococcus pneumoniae

<400> 4
ttgagaccgt ctttacagtc ctt 23

<210> 5
<211> 23
<212> DNA
<213> Streptococcus pyogenes

<400> 5
gttttgcctc tcatattaaa gtc 23

<210> 6
<211> 23
<212> DNA
<213> Streptococcus pyogenes

<400> 6
tctttattga agcagataat tcc 23

<210> 7
<211> 23
<212> DNA
<213> Streptococcus pyogenes

<400> 7
cgttgaaaca gtttttacag tct 23

<210> 8
<211> 23
<212> DNA
<213> Chlamydia pneumoniae

<400> 8
ggtcttcatc atctagtcta tga 23

<210> 9
<211> 23
<212> DNA
<213> Chlamydia pneumoniae

<400> 9
ggttgtagac wacagcattg acg 23

<210> 10
<211> 23
<212> DNA
<213> Chlamydia pneumoniae

<400> 10
agccatggca gsttattgct cta 23

<210> 11
<211> 23
<212> DNA
<213> Chlamydia pneumoniae

<400> 11
ggattgatgt tsgcatttta gag 23

<210> 12
<211> 23
<212> DNA
<213> Chlamydia pneumoniae

<400> 12
gggtattgtc wtcgtagata atg 23

<210> 13
<211> 22
<212> DNA
<213> Chlamydia pneumoniae

<400> 13
ttattgctct aggattgatg tt 22

<210> 14
<211> 23
<212> DNA
<213> Chlamydia pneumoniae

<400> 14
gcattttaga gsacgggggt att 23

<210> 15
<211> 24

<212> DNA
<213> Mycoplasma pneumoniae

<400> 15
tcaaactaac ccttaaagac aact 24

<210> 16
<211> 23
<212> DNA
<213> Mycoplasma pneumoniae

<400> 16
tgaaacagtg tttacggtac tcc 23

<210> 17
<211> 23
<212> DNA
<213> Haemophilus influenzae

<400> 17
atgataattc tgtatcggtg caa 23

<210> 18
<211> 23
<212> DNA
<213> Haemophilus influenzae

<400> 18
agcagaagtt attatgactg tgc 23

<210> 19
<211> 23
<212> DNA
<213> Haemophilus influenzae

<400> 19
gacgataact cttataaagt atc 23

<210> 20
<211> 24
<212> DNA
<213> Haemophilus influenzae

<400> 20
gataccgatg acggtactgg tttg 24

<210> 21
<211> 23
<212> DNA
<213> Staphylococcus aureus

<400> 21
agtgtgggaa attgtcgata ata 23

<210> 22
<211> 25
<212> DNA
<213> Staphylococcus aureus

<400> 22
tgaagttgtt attgaaaaag ataac 25

<210> 23
<211> 23
<212> DNA
<213> Staphylococcus aureus

<400> 23
ggattaaagt aacggataac gga 23

<210> 24
<211> 24
<212> DNA
<213> Staphylococcus aureus

<400> 24
ctgtcgaagt tattttaact gttt 24

<210> 25
<211> 23
<212> DNA
<213> Staphylococcus aureus

<400> 25
cgacttcaga gagaggtttg cac 23

<210> 26
<211> 23
<212> DNA
<213> Pseudomonas aeruginosa

<400> 26
gaaatcagca tcaccatcca tac 23

<210> 27
<211> 23
<212> DNA
<213> Pseudomonas aeruginosa

<400> 27
atacggatga gtcgatcact gtc 23

<210> 28
<211> 23
<212> DNA
<213> Pseudomonas aeruginosa

<400> 28
gacgacaaca cctacaaggt gtc 23

<210> 29
<211> 24
<212> DNA
<213> Pseudomonas aeruginosa

<400> 29
gacaccgacg atggcaccgg tctg 24

<210> 30
<211> 23
<212> DNA
<213> Neisseria gonorrhoeae

<400> 30
ttcgacaaca acagctacaa aat 23

<210> 31
<211> 23
<212> DNA
<213> Neisseria gonorrhoeae

<400> 31
atatggtgtt tgaagtattg gac 23

<210> 32
<211> 23
<212> DNA
<213> Neisseria gonorrhoeae

<400> 32
gacaaaatca cggtaacgat aca 23

<210> 33
<211> 23
<212> DNA
<213> Neisseria gonorrhoeae

<400> 33
gacaacaaca gctacaaaat ctc 23

<210> 34
<211> 23
<212> DNA
<213> Escherichia coli

<400> 34
tattcgaggt ggtagataac gct 23

<210> 35
<211> 23
<212> DNA
<213> Escherichia coli

<400> 35
ttcacgccga taactctgtc tct 23

<210> 36
<211> 23
<212> DNA
<213> Escherichia coli

<400> 36
cgccgataac tctgtctctg tac 23

<210> 37
<211> 23

<212> DNA
<213> Escherichia coli

<400> 37
gacgataact cctataaagt gtc 23

<210> 38
<211> 24
<212> DNA
<213> Escherichia coli

<400> 38
gacacggatg acggcaccgg tctg 24

<210> 39
<211> 23
<212> DNA
<213> Moraxella catarrhalis

<400> 39
agctgccgag gttattatga cgg 23

<210> 40
<211> 23
<212> DNA
<213> Moraxella catarrhalis

<400> 40
gatgataatt catacaaagt atc 23

<210> 41
<211> 23
<212> DNA
<213> Moraxella catarrhalis

<400> 41
tgtggatatc caccctgaag aag 23

<210> 42
<211> 23
<212> DNA
<213> Moraxella catarrhalis

<400> 42
ataccgatga tggtacaggc ttg 23

<210> 43
<211> 23
<212> DNA
<213> Legionella pneumophila

<400> 43
gatgataatt cctacaaggt atc 23

<210> 44
<211> 23
<212> DNA
<213> Legionella pneumophila

<400> 44
agcagccgaa gtcatcatga cag 23

<210> 45
<211> 23
<212> DNA
<213> Legionella pneumophila

<400> 45
tgtagatatt cataaagaag aag 23

<210> 46
<211> 23
<212> DNA
<213> Legionella pneumophila

<400> 46
atacagatga tggaaccggt ttg 23

<210> 47
<211> 23
<212> DNA
<213> Legionella pneumophila

<400> 47
cagatgatgg aaccggtttg cat 23

<210> 48
<211> 23
<212> DNA
<213> Fusobacterium necrophorum

<400> 48
caacatcagc caggggatta cat 23

<210> 49
<211> 23
<212> DNA
<213> Fusobacterium necrophorum

<400> 49
ggaattccag tagacataca tcc 23

<210> 50
<211> 24
<212> DNA
<213> Fusobacterium necrophorum

<400> 50
tgaaaatgat aactataaag tgtc 24

<210> 51
<211> 23
<212> DNA
<213> Staphylococcus aureus

<400> 51
cggtaacgaa atagatgtaa caa 23

<210> 52
<211> 23
<212> DNA
<213> Staphylococcus aureus

<400> 52
agaagataat ggacgtggta tgc 23

<210> 53
<211> 23
<212> DNA
<213> Staphylococcus aureus

<400> 53
gtaaaccgac agtcgaagtt atc 23

<210> 54
<211> 24
<212> DNA
<213> Staphylococcus aureus

<400> 54
caactgataa acggggatta catc 24

<210> 55
<211> 23
<212> DNA
<213> Staphylococcus aureus

<400> 55
ggacaaggcg gctataaaac ttc 23

<210> 56
<211> 23
<212> DNA
<213> Streptococcus pyogenes

<400> 56
tggagatgat attaaggttg tta 23

<210> 57
<211> 23
<212> DNA
<213> Streptococcus pyogenes

<400> 57
gtcagtgtgg cagatagcgg acg 23

<210> 58
<211> 23
<212> DNA
<213> Streptococcus pyogenes

<400> 58
ggattcccac cgttcaagtt att 23

<210> 59
<211> 23

<212> DNA
<213> Streptococcus pyogenes

<400> 59
caacagatgc tacgggattg cac 23

<210> 60
<211> 23
<212> DNA
<213> Streptococcus pyogenes

<400> 60
ggtcagggtg gctacaaaac gtc 23

<210> 61
<211> 23
<212> DNA
<213> Streptococcus pneumoniae

<400> 61
tggtgatcgt attgatgtaa cta 23

<210> 62
<211> 23
<212> DNA
<213> Streptococcus pneumoniae

<400> 62
ctaacggttc aagaccatgg acg 23

<210> 63
<211> 23
<212> DNA
<213> Streptococcus pneumoniae

<400> 63
gaattccaac tgttgaggtt atc 23

<210> 64
<211> 23
<212> DNA
<213> Streptococcus pneumoniae

<400> 64
cgaccgatgg cgctggtctt cat 23

<210> 65
<211> 23
<212> DNA
<213> Streptococcus pneumoniae

<400> 65
ggtcaaggtg gctataagac atc 23

<210> 66
<211> 23
<212> DNA
<213> Mycoplasma pneumoniae

<400> 66
tgctaatact attgccgttg ttt 23

<210> 67
<211> 23
<212> DNA
<213> Mycoplasma pneumoniae

<400> 67
ataactgtta gtgacaacgg tcg 23

<210> 68
<211> 23
<212> DNA
<213> Mycoplasma pneumoniae

<400> 68
agatttctac gattgacacc gtc 23

<210> 69
<211> 23
<212> DNA
<213> Mycoplasma pneumoniae

<400> 69
gataacgatt cttataagat tgc 23

<210> 70
<211> 294
<212> DNA
<213> Moraxella catarrhalis

<400> 70

cgaccaggga tgtatattgg tgataccgat gatggtacag gcttgcacca tatggtgttt 60

gaggtggtgg ataatgccat tgatgaggca ttggcaggtc actgtgatga gattaatatt 120

atcgtccatg acgatgaatc tgtttcggtg atggactatg ggcgtggtat tcctgtggat 180

atccaccctg aagaaggtgt atcagctgcc gaggttatta tgacggtgct tcatgcaggc 240

ggtaaatttg atgataattc atacaaagta tctgggggcc tgcacggcgt agga 294

<210> 71
<211> 257
<212> DNA
<213> Legionella pneumophila

<400> 71

ggggatacag atgatggaac cggtttgcat cacatggttt ttgaggttgt agataattca 60

atagatgagt ctctggcagg atattgcaag gaaattttg ttaccatcca tagcgatgag 120

tcaattacag ttaaggacga tggccgtggt attcctgtag atattcataa agaagaaggc 180

aaatcagcag ccgaagtcat catgacagtc ctacatgctg gaggtaaatt tgatgataat 240

tcctacaagg tatctgg 257

<210> 72
<211> 290
<212> DNA
<213> Fusobacterium necrophorum

<400> 72

cgcccaggga tgtacatcgg aacaacatca gccaggggat tacatcattt agtatgggaa 60

gtggtagata attctgtgga tgaagcattt gctgggtatt gtaataggat tactgtaagt 120

attttgcctg ataacattat tcaagtagag gataatggaa gaggaattcc agtagacata 180

catccaaaat atggaaaatc cgctttggaa attgtattga cggtattaca tgctggggga 240

aaatttgaaa atgataacta taaagtgtca ggtggactgc acggagttgg 290

<210> 73
<211> 291
<212> DNA
<213> Streptococcus mitis

<220>
<221> misc_feature
<222> (18)..(18)
<223> n is a, t, c, or g

<220>
<221> misc_feature
<222> (288) .. (288)
<223> n is a, t, c, or g

<400> 73

agtcccggga tgtacatngg gtcaacttca aaagaaggtc ttcaccatct agtctgggaa 60

attgttgata actcaattga cgaggccttg gcaggatttg ctagccatat tcaagtcttt 120

attgagccag ataattcgat tacagttgtt gatgatggac gtggtatccc agtcgatatt 180

caggaaaaaa caggtcgacc tgccgttgag actgtcttta ctgttcttca cgctggagga 240

aagtttggcg gtggcggata taaggtctca gacggtcttc atggcgtngg a 291

<210> 74

<211> 288
<212> DNA
<213> Streptococcus oralis

<400> 74

agaccgggga tgtatattgg atcgaccgac ggtgctggtc tccatcatct agtctgggaa 60

atcgtggata atgcggttga cgaagccttg tctggatttg gtgatcgcat cgatgtgacg 120

attaataagg acgggagttt aacggttcaa gaccacggac gtgggatgcc aacgggaatg 180

cacgccatgg gaattccaac tgttgaagtt atctttacca ttctccacgc tggagggaaa 240

ttcggtcaag gtggctataa gacatctggt ggtctgcatg gggttgga 288

<210> 75
<211> 294
<212> DNA
<213> Haemophilus parainfluenzae

<400> 75

agaccgggga tgtatatcgg ggataccgat gatggaacag gcctacacca tatggtgttt 60

gaggtggtgg ataacgctat cgatgaagcg cttgctggct attgttccga tattatcgtc 120

actattcatg atgataattc tgtttccgta caagatgacg gccgcggtat tccggtagat 180

attcacccag aagaaggggt ttctgcggca gaagcaatca tgacagtact tcacgcaggt 240

ggtaaattcg atgataactc ttataaagta tcagggggac tacacggcgt tgga 294

<210> 76
<211> 20
<212> DNA
<213> Artificial Sequence

<220>

<223> primer

<220>
<221> misc feature
<222> (6) .. (6)
<223> w is a or t

<220>
<221> misc_feature
<222> (9)..(9)
<223> k is g or t

<220>
<221> misc_feature
<222> (15)..(15)
<223> y is c or t

<220>

<221> misc_feature
<222> (18)..(18)
<223> h is a, c or t

<400> 76
cgtccwggka tgtayathgg 20

<210> 77
<211> 20
<212> DNA
<213> Artificial Sequence

<220>
<223> primer

<220>
<221> misc_feature
<222> (3) .. (3)
<223> h is a, c or t

<220>
<221> misc_feature
<222> (6)..(6)
<223> r is a or g

<220>
<221> misc feature
<222> (9)..(9)
<223> r is a or, g

<220>
<221> misc_feature

<222> (12)..(12)
<223> w is a or t

<220>
<221> misc_feature
<222> (15)..(15)
<223> w is a or t

<220>
<221> misc_feature
<222> (18)..(18)
<223> d is a, g or t

<400> 77
cchacrccrt gwaawccdcc 20